(19) Europäisches Patentamt
European Patent Office
Office européen des brevets

(11) EP 1 495 757 A1

(12) **EUROPEAN PATENT APPLICATION**
published in accordance with Art. 158(3) EPC

(43) Date of publication:
**12.01.2005 Bulletin 2005/02**

(21) Application number: **03746165.4**

(22) Date of filing: **02.04.2003**

(51) Int Cl.[7]: **A61K 31/4406**, A61K 31/443, A61K 31/44, A61K 31/4402, A61K 31/4409, A61P 1/04, A61P 11/06, A61P 11/08, A61P 17/00, A61P 19/02, A61P 25/18, A61P 25/24, A61P 27/02, A61P 27/16, A61P 29/00, A61P 31/04, A61P 37/08, A61P 43/00, A61P 17/04
// (C07D213/56, 213:57, 213:59, 405:12, 213:61, 213:80)

(86) International application number:
**PCT/JP2003/004227**

(87) International publication number:
**WO 2003/086396 (23.10.2003 Gazette 2003/43)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **02.04.2002 JP 2002099491**

(71) Applicant: **TSUMURA & CO.**
**Chuo-ku, Tokyo 103-0027 (JP)**

(72) Inventors:
- **HATTORI, Tomohisa, c/o Tsumura & Co. Ibaraki 300-1192 (JP)**
- **SASAKI, Toshinobu, c/o Tsumura & Co. Ibaraki 300-1192 (JP)**
- **HASEGAWA, Yoshihiro, c/o Tsumura & Co. Ibaraki 300-1192 (JP)**
- **OBATA, Tatsuhiro, c/o Tsumura & Co. Ibaraki 300-1192 (JP)**

(74) Representative:
**Ziebig, Marlene, Dr. Dipl.-Chem. et al**
**Anwaltskanzlei**
**Gulde Hengelhaupt Ziebig & Schneider**
**Wallstrasse 58/59**
**10179 Berlin (DE)**

(54) **PHOSPHODIESTERASE IV INHIBITOR CONTAINING PYRIDYLACRYLAMIDE DERIVATIVE**

(57) This invention relates to a phosphodiesterase IV inhibitor containing as an active ingredient a pyridylacrylamide derivative represented by the following formula (I):

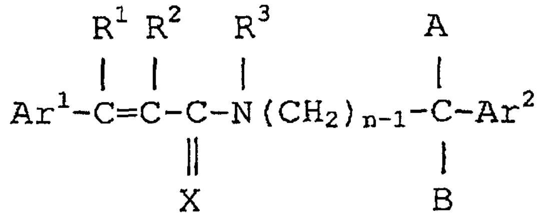

(I)

(wherein $Ar^1$ represents substituted or unsubstituted pyridyl; $Ar^2$ represents phenyl substituted by alkoxy, etc.; $R^1$ represents hydrogen, alkyl, or aryl; $R^2$ represents hydrogen, alkyl, cyano, or alkoxycarbonyl; $R^3$ represents hydrogen or

EP 1 495 757 A1

optionally substituted alkyl; X represents oxygen or sulfur; A and B are the same or different and each independently represents hydrogen, hydroxyl, alkoxy, or alkylthio, or A and B together represent oxo, thioxo, etc., or A may be hydroxyl and B may be 1-alkylimidazol-2-yl; and n is an integer from 1 to 3) or a pharmaceutically acceptable salt thereof.

## Description

Technical Field

The present invention relates to a phosphodiesterase IV inhibitor containing a pyridylacrylamide derivative.

Background Art

The intracellular second messengers CAMP and cGMP are degraded by phosphodiesterase (PDE), which is classified into at least types I to VII, and inactivated. PDE is widely distributed in each tissue and organ in vivo. Among these PDEs, PDE IV is a substance which selectively degrades CAMP, and which is typically present in central tissue as well as in various organs such as heart, lung, kidney, and various blood cell components. It is also known to be involved in the induction of various cytokines such as IL-1, IL-6, and TNF-α.

The use and development of catechol derivatives typically represented by rolipram that has a selective inhibitory action against PDE IV, or quinazoline derivatives typically represented by nitraquazone, xanthine-type derivatives typically represented by theophylline and denbufylline, and the like as antidepressants, antiasthmatic agents, anti-inflammatory agents and the like is proceeding.

Meanwhile, in WO 99/05109 it is disclosed that a pyridylacrylamide derivative represented by the following formula (A) or a pharmaceutically acceptable salt thereof is useful as a therapeutic agent for nephritis and a TGF-β inhibitor, however there is no reference to an activity thereof against PDE IV:

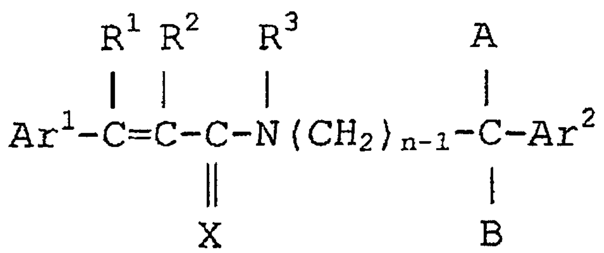

(A)

[wherein $Ar^1$ represents a substituted or unsubstituted pyridyl group; $Ar^2$ represents a substituted or unsubstituted phenyl group; $R^1$ represents a hydrogen atom, a $C_{1-6}$ alkyl group, or an aryl group; $R^2$ represents a hydrogen atom, a $C_{1-6}$ alkyl group, a cyano group, or a $C_{1-6}$ alkoxy-carbonyl group; $R^3$ represents a hydrogen atom or an optionally substituted $C_{1-6}$ alkyl group; X represents an oxygen atom or a sulfur atom; A and B are the same or different from each other, and each independently represents a hydrogen atom, a hydroxyl group, a $C_{1-6}$ alkoxy group or a $C_{1-6}$ alkylthio group, or A and B together represent an oxo-group, a thioxo group, a group represented by the following formula:

$$= N\text{-}Y$$

(wherein Y represents a di-($C_{1-6}$ alkyl) amino group, a hydroxyl group, an aralkyloxy group, or a $C_{1-6}$ alkoxy group), or a group represented by the following formula:

$$-Z^1\text{-}M\text{-}Z^2\text{-}$$

(wherein $Z^1$ and $Z^2$ are the same or different from each other, and each independently represents an oxygen atom, a sulfur atom or an imino group that is optionally substituted with a $C_{1-6}$ alkyl group, and M represents an alkylene group having 2 to 4 chain members or a 1,2-phenylene group),

or A may be a hydroxyl group and then B may be a 1- $C_{1-6}$ alkyl-imidazol-2-yl group;

and n represents an integer from 1 to 3.]

Further, in WO 93/04035 (JP Patent Publication (Kohyo) No. 6-510030 A (1994)) a pyridylacrylamide derivative represented by the following formula (B):

$$Ar^3\text{-}CH = CH\text{-}CO\text{-}NHCH_2\text{-}Ar^4 \quad (B)$$

(wherein $Ar^3$ represents a 3-pyridyl group, and $Ar^4$ represents a 3,5-di-tert-butyl-4-hydroxyphenyl group) is described as one specific example of a number of 3,5-di-tert- butyl-4-hydroxyphenyl derivatives. The compound in question is disclosed as being useful as a therapeutic agent for metabolic disease, such as an anti-atherosclerosis drug, and is also disclosed as having cytoprotective and anti-inflammatory activity as well as antiasthmatic activity, however there is no reference to an activity thereof against PDE IV.

Disclosure of the Invention

It is an object of the present invention to provide a novel type of phosphodiesterase IV inhibitor.
The present inventors conducted concentrated exploratory research with the intention of developing a phosphodiesterase IV inhibitor, and as a result found that among the pyridylacrylamide derivatives disclosed in WO 99/05109, some of the compounds have excellent inhibitory action against phosphodiesterase IV, thus completing the present invention.
More specifically, the present invention comprises the following:

(1) A phosphodiesterase IV inhibitor containing as an active ingredient a pyridylacrylamide derivative represented by the following formula (I):

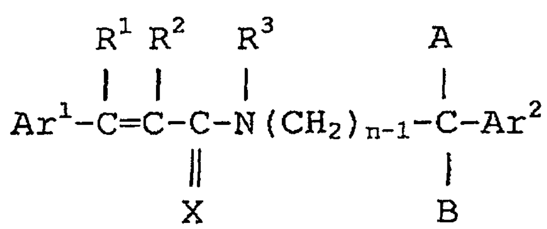
(I)

wherein

$Ar^1$ represents a substituted or unsubstituted pyridyl group;
$Ar^2$ represents a substituted phenyl group that is substituted with at least 1 to 3 substituents selected from the group consisting of a $C_{1-6}$ alkoxy group, a $C_{2-6}$ alkenyloxy group, an aralkyloxy group, and an aryloxy group;
$R^1$ represents a hydrogen atom, a $C_{1-6}$ alkyl group, or an aryl group;
$R^2$ represents a hydrogen atom, a $C_{1-6}$ alkyl group, a cyano group, or a $C_{1-6}$ alkoxy-carbonyl group;
$R^3$ represents a hydrogen atom or an optionally substituted $C_{1-6}$ alkyl group;
X represents an oxygen atom or a sulfur atom;
A and B are the same or different from each other, and each independently represents a hydrogen atom, a hydroxyl group, a $C_{1-6}$ alkoxy group or a $C_{1-6}$ alkylthio group, or A and B together represent an oxo group, a thioxo group, a group represented by the following formula:

$$= N\text{-}Y$$

wherein Y represents a di-($C_{1-6}$ alkyl) amino group, a hydroxyl group, an aralkyloxy group, or a $C_{1-6}$ alkoxy group or a group represented by the following formula:

$$- Z^1\text{-}M\text{-}Z^2\text{-}$$

wherein $Z^1$ and $Z^2$ are the same or different from each other, and each independently represents an oxygen atom, a sulfur atom, or an imino group that may be optionally substituted with a $C_{1-6}$ alkyl group; and M represents an alkylene group having 2 to 4 chain members or a 1,2-phenylene group, or
A may be a hydroxyl group and B may be a 1- $C_{1-6}$-alkyl-imidazol-2-yl group; and
n represents an integer from 1 to 3,

or a pharmaceutically acceptable salt thereof.

(2) The phosphodiesterase IV inhibitor described in the above (1), wherein in the above formula (I), $Ar^1$ represents a substituted or unsubstituted pyridyl group; $Ar^2$ represents a substituted phenyl group that is substituted with at least 1 to 3 substituents selected from the group consisting of a $C_{1-6}$ alkoxy group, a $C_{2-6}$ alkenyloxy group, an aralkyloxy group, and an aryloxy group; $R^1$ represents a hydrogen atom, a $C_{1-6}$ alkyl group, or an aryl group; $R^2$ represents a hydrogen atom, a methyl group, a cyano group, or a $C_{1-6}$ alkoxy-carbonyl group; $R^3$ represents a hydrogen atom or an optionally substituted $C_{1-3}$ alkyl group; X represents an oxygen atom or a sulfur atom; A and B each independently represents a hydrogen atom, or A and B together represent an oxo group; provided that when A and B each independently represents a hydrogen atom, then n represents 1 or 2, and when A and B together represent an oxo group, then n represents 2.

(3) The phosphodiesterase IV inhibitor described in the above (2), wherein in the above formula (I), $Ar^2$ represents a substituted phenyl group that is substituted with 1 to 3 $C_{1-6}$ alkoxy groups, and $R^3$ represents a $C_{1-3}$ alkyl group.

(4) The phosphodiesterase IV inhibitor described in any of the above (1) to (3), wherein in the above formula (I), a substituted phenyl group represented by $Ar^2$ is further substituted with at least one member selected from the group consisting of a halogen atom, a hydroxyl group, an optionally substituted amino group, a substituted $C_{1-6}$ alkoxy group, an optionally substituted $C_{1-6}$ alkyl group, an aryl group, a $C_{1-6}$ alkylthio group, a carboxyl group, a $C_{1-6}$ alkoxy-carbonyl group, a sulfamoyl group, and group -O-CO-$R^4$ (where $R^4$ represents a $C_{1-6}$ alkyl group, an aryl group, a $C_{1-6}$ alkoxy group, or an optionally substituted amino group).

(5) The phosphodiesterase IV inhibitor described in any of the above (1) to (4), which is a preventive or therapeutic agent for a phosphodiesterase IV-involving disease selected from the group consisting of bronchial asthma, chronic bronchitis, atopic dermatitis, hives, allergic rhinitis, conjunctivitis, rheumatoid arthritis (chronic articular rheumatism), gonarthrosis, septicemia, ulcerative colitis, manic-depressive psychosis, schizophrenia and Crohn's disease.

In the above formula (I), examples of a pyridyl group represented by $Ar^1$ include a 2-pyridyl group, a 3-pyridyl group and a 4-pyridyl group, and the 2-pyridyl group, the 3-pyridyl group and the 4-pyridyl group may be substituted by at least one member selected from the group consisting of, for example, a halogen atom, a $C_{1-6}$ alkyl group, a $C_{1-6}$ alkoxy group, and a $C_{1-6}$ alkoxy-carbonyl group.

In the above formula (I), a substituted phenyl group represented by $Ar^2$ has at least 1 to 3 substituents selected from the group consisting of a $C_{1-6}$ alkoxy group, a methylenedioxy group, a $C_{2-6}$ alkenyloxy group, an aralkyloxy group, and an aryloxy group, and depending on a case, may have one or more substituents selected from the group consisting of a halogen atom, a hydroxyl group, an optionally substituted amino group, a substituted $C_{1-6}$ alkoxy group, an optionally substituted $C_{1-6}$ alkyl group, an aryl group, a $C_{1-6}$ alkylthio group, a carboxyl group, a $C_{1-6}$ alkoxy-carbonyl group, a sulfamoyl group, and group -O-CO-$R^4$ (where $R^4$ represents a $C_{1-6}$ alkyl group, an aryl group, a $C_{1-6}$ alkoxy group, or an optionally substituted amino group).

In the present specification, a $C_{1-6}$ alkyl group and a "$C_{1-6}$ alkyl" of each substituent may be either linear, branched, or cyclic ($C_{3-6}$ cycloalkyl), and examples thereof include a methyl group, an ethyl group, a propyl group, an isopropyl group, a butyl group, an isobutyl group, a sec-butyl group, a tert-butyl group, a pentyl group, an isopentyl group, a hexyl group, a cyclopentyl group and a cyclohexyl group. As examples of a $C_{1-6}$ alkoxy group and "$C_{1-6}$ alkoxy" in each substituent, all alkoxy groups that can be derived from the above $C_{1-6}$ alkyl group may be mentioned, and examples thereof include a methoxy group, an ethoxy group, a propoxy group, an isopropoxy group, a butoxy group, an isobutoxy group, a sec-butoxy group, a tert-butoxy group, a pentyloxy group, an isopentyloxy group, a hexyloxy group, a cyclopentyloxy group and a cyclohexyloxy group. Among these, most preferably a $C_{1-6}$ alkyl group is a methyl group and a $C_{1-6}$ alkoxy group is a methoxy group.

As examples of a $C_{1-6}$ alkylthio group, all $C_{1-6}$ alkylthio groups that can be derived from the above $C_{1-6}$ alkyl group may be mentioned, and examples thereof include a methylthio group, an ethylthio group, a propylthio group, an isopropylthio group, a butylthio group, an isobutylthio group, a sec-butylthio group, a tert-butylthio group, a pentylthio group, an isopentylthio group, a hexylthio group, a cyclopentylthio group and a cyclohexylthio group.

Examples of a di-($C_{1-6}$ alkyl) amino group include a dimethylamino group and a diethylamino group.

An Example of a 1-$C_{1-6}$ alkyl-imidazol-2-yl group includes a 1-methylimidazol-2-yl group.

As examples of a $C_{1-6}$ alkoxy-carbonyl group, all alkoxycarbonyl groups that can be derived from the above $C_{1-6}$ alkoxy group may be mentioned, and examples thereof include a methoxycarbonyl group, an ethoxycarbonyl group, an isopropoxycarbonyl group, and a butoxycarbonyl group.

Examples of an aryl group include an optionally substituted phenyl group, such as a phenyl group or a p-methoxyphenyl group. Examples of an aryloxy group include an optionally substituted phenoxy group, such as a phenoxy group or a p-methylphenoxy group. Examples of an aralkyloxy group include an optionally substituted benzyloxy group.

Examples of a halogen atom include a fluorine atom, a chlorine atom, a bromine atom, and an iodine atom.

Examples of a $C_{2-6}$ alkenyl-oxy group include an allyloxy group and an isobutenyloxy group, and examples

of a $C_{1-6}$ alkylthio group include a methylthio group and an ethylthio group.

Examples of group -O-CO-$R^4$ include an acetoxy group, an isobutyryloxy group, a pivaloyloxy group, a benzoyloxy group, an ethoxycarbonyloxy group, an ethylcarbamoyloxy group and a dimethylcarbamoyloxy group.

A $C_{1-6}$ alkyl group represented by $R^3$ and a $C_{1-6}$ alkyl group as a substituent on a substituted phenyl group represented by $Ar^2$ may be substituted by a suitable substituent, for example, at least one member selected from the group consisting of a $C_{1-6}$ alkoxy-carbonyl group and a halogen atom. Examples of the above substituted $C_{1-6}$ alkyl group include a methoxycarbonylmethyl group and a trifluoromethyl group.

As a substituted $C_{1-6}$ alkoxy group as a substituent on a substituted phenyl group represented by $Ar^2$, a $C_{1-6}$ alkoxy group substituted with a suitable substituent, for example, at least one member selected from the group consisting of a $C_{1-6}$ alkoxy group, a $C_{1-6}$ alkoxy-$C_{1-6}$ alkoxy group, an aryl group, a carboxyl group, a $C_{1-6}$ alkoxy-carbonyl group, an aralkyloxycarbonyl group, a halogen atom, and group -CONR$^5$R$^6$ (wherein, $R^5$ and $R^6$ are the same or different from each other, and represent a hydrogen atom, an optionally substituted $C_{1-6}$ alkyl group, an optionally substituted $C_{1-6}$ alkoxy group, or a hydroxyl group, and further, $R^5$ and $R^6$may bind to each other to form a ring together with a nitrogen atom binding thereto), may be mentioned. Examples of the above substituted $C_{1-6}$ alkoxy group include a methoxymethoxy group, a (2-methoxyethoxy)methoxy group, a carboxymethoxy group, a methoxycarbonylmethoxy group, an ethoxycarbonylmethoxy group, an isopropoxycarbonyl-methoxy group, a tert-butoxycarbonylmethoxy group, a 1-(ethoxycarbonyl)isopropoxy group, a 3-(ethoxycarbonyl)propoxy group, a benzyloxycarbonylmethoxy group, a trifluoromethoxy group, a (methylcarbamoyl)methoxy group, a (dimethylcarbamoyl)methoxy group, a (3-pyridylmethylcarbamoyl)methoxy group, an (ethylcarbamoyl)methoxy group, a (diethylcarbamoyl)methoxy group, a (hexylcarbamoyl)methoxy group, a (2-methoxyethyl)-carbamoylmethoxy group, a (2-benzylthioethyl)carbamoylmethoxy group, a (propylcarbamoyl)methoxy group, an (isopropylcarbamoyl)methoxy group, a (methylmethoxycarbamoyl)methoxy group, an (ethoxycarbonylmethylcarbamoyl) methoxy group, a (cyclopentylcarbamoyl)methoxy group and a morpholinocarbonyl methoxy group.

When a substituent on a substituted phenyl group represented by $Ar^2$ is a $C_{1-6}$ alkoxy group, a substituted $C_{1-6}$ alkoxy group, or an optionally substituted $C_{1-6}$ alkyl group, and two or more substituents are present, the two groups may bind through an alkyl moiety to form an alkylene group such as a tetramethylene group or a trimethylene group, or an alkylenedioxy group such as a methylenedioxy group. Further, these alkylene groups or alkylenedioxy groups may be optionally substituted with a suitable substituent such as, for example, a $C_{1-6}$ alkoxy-carbonyl group such as an ethoxycarbonyl group.

An amino group as a substituent on a substituted phenyl group represented by $Ar^2$, and an amino group represented by $R^4$ in the above group -O-CO-$R^4$ may be substituted with a suitable substituent, for example, at least one member selected from the group consisting of an optionally substituted $C_{1-6}$ alkyl group and an optionally substituted $C_{1-6}$ alkoxy group, and may also be cyclic. Examples of the above substituted amino group include a methylamino group, a dimethylamino group, a 3-pyridylmethylamino group, an ethylamino group, a diethylamino group, a (2-methoxyethyl)amino group, a (2-benzylthioethyl)amino group, a propylamino group, an isopropylamino group, a cyclopentylamino group, a hexylamino group, an ethoxycarbonylmethylamino group, a methylmethoxy amino group, a hydroxyamino group, and a morpholino group.

An alkylene group represented by M is an alkylene group having from 2 to 4 chain members, that is, the number of carbon atoms constituting the alkylene chain is between 2 and 4, and the alkylene group may have between 1 and 4 side chains having 1 to 3 carbon atoms, such as a methyl group, an ethyl group, and a propyl group.

The compound shown by the above formula (I) is a known compound that is disclosed in WO 99/05109.

Examples of a pharmaceutically acceptable salt of the compound shown by the above formula (I) include an inorganic salt such as hydrochloride, sulfate, hydrobromide, nitrate and phosphate, and an organic salt such as trifluoroacetate, tartrate, citrate, malate, maleate, fumarate, methanesulfonate, benzenesulfonate and toluenesulfonate. Depending on a compound, there are cases where a hydrate may be formed, and the use of these hydrates also falls within the scope of the present invention.

Further, a compound represented by the above formula (I) comprises cis-trans stereoisomers, as is apparent from the formula of the chemical structure thereof. The use of these isomers also falls within the scope of the present invention.

A compound represented by the above formula (I) can be produced by a variety of methods. As typical methods for producing the compound, the methods described in the following (1) to (8) can be exemplified.

(1) In formula (I), when $R^2$ is a hydrogen atom or a $C_{1-6}$ alkyl group, and X is an oxygen atom, a compound (I) can be produced through amidation by reacting a carboxylic acid represented by the following general formula (II):

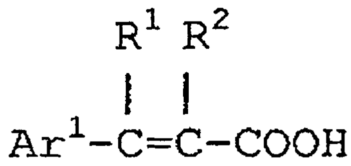

(II)

(wherein $Ar^1$, $R^1$, and $R^2$ have the aforesaid meaning) or a reactive derivative thereof, with an amine represented by the following general formula (III):

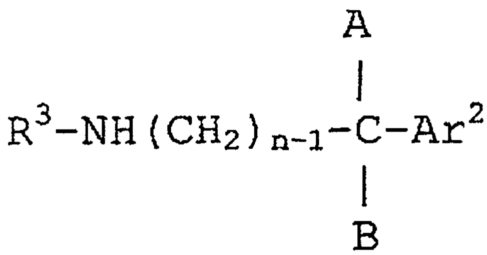

(III)

(wherein $Ar^2$, $R^3$, A, B and n have the aforesaid meaning).

The pyridylacrylic acid derivative (II) and the amine compound (III), which are the starting materials, are compounds that are commercially available or that can be obtained by a common method.

This reaction, in particular when providing compound (II) for reaction in the form of carboxylic acid, is preferably conducted in the presence of a condensing agent (for example, dicyclohexylcarbodiimide, N,N'-carbonyldiimidazole, 1-hydroxybenzotriazole, N-hydroxysuccinimide, diethyl phosphorocyanidate, diphenoxyphosphoryl azide, or pivaloyl chloride), and use of the above diethyl phosphorocyanidate together with triethylamine is particularly advantageous. Examples of a reactive derivative of compound (II) include acid anhydride, mixed acid anhydride, and the like.

The reaction is conducted using an appropriate solvent that does not participate in the reaction, for example, an organic solvent such as tetrahydrofuran, N,N-dimethylformamide or dichloromethane, and conducting the reaction under anhydrous conditions is particularly preferable. A reaction temperature is not particularly limited, and normally a temperature obtained by ice-cooling to around room temperature can be employed. A reaction time is normally from 0.5 to 20 hours, and a desired material may be isolated by a standard method in the art after completion of the reaction.

(2) When, in formula (I), $R^2$ is a cyano group or a $C_{1-6}$ alkoxy-carbonyl group and X is an oxygen atom, compound (I) can be produced by subjecting a nicotinaldehyde derivative represented by the following general formula (IV):

$$Ar^1\text{-CHO} \qquad (IV)$$

(wherein $Ar^1$ has the same meaning as described above) and an active methylene compound represented by the following general formula (V):

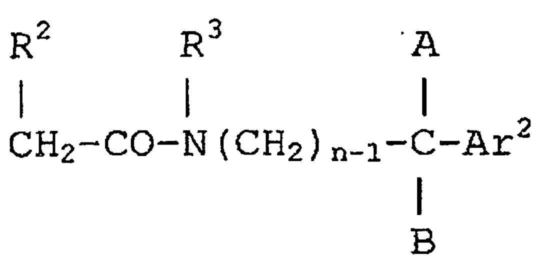

(V)

(wherein $R^2$ is a cyano group or a $C_{1-6}$ alkoxy-carbonyl group, and $Ar^2$, $R^3$, A, B and n have the aforesaid meaning)

to a Knoevenagel condensation reaction in the presence of a base catalyst.

The nicotinaldehyde derivative (IV) and the active methylene compound (V), which are the starting materials, are compounds that are commercially available or that can be obtained by a conventional method.

For this reaction, an appropriate solvent that does not participate in the reaction can be used, for example, an organic solvent such as benzene, toluene or ethanol, and examples of a base catalyst that can be used for the reaction include pyridine and piperidine. A reaction temperature is from 80 to 140°C, and a desired material can be isolated by a standard method in the art after completion of the reaction.

(3) When, in formula (I), X is a sulfur atom, compound (I) can be produced through thionization by reacting a compound obtained by the above method (1), more specifically, an amide represented by the following general formula (VI):

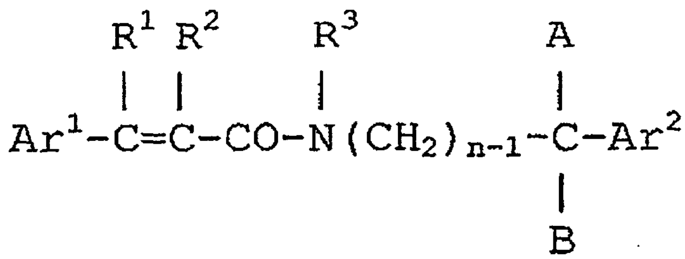

(VI)

wherein, $Ar^1$, $Ar^2$, $R^1$, $R^2$, $R^3$, A, B and n have the aforesaid meaning,
with a sulfurizing agent such as Lawesson's reagent.

At such time, a solvent that does not participate in the reaction, such as toluene or xylene, can be used, and a reaction temperature is normally between 110 and 140°C. After completion of the reaction, a desired material can be isolated by a standard method in the art.

(4) A compound of formula (I) in which X is an oxygen atom and a substituted phenyl group represented by $Ar^2$ is substituted by at least one member of the group consisting of group $-OC(R^7)_2COR^8$ (where $R^7$ represents a hydrogen atom or a methyl group, and $R^8$ represents a hydroxyl group, a $C_{1-6}$ alkoxy group, or an optionally substituted amino group) and group $-O-CO-R^4$ (where $R^4$ has the same meaning as described above) can be produced by introducing group $-OC(R^7)_2COR^8$ or group $-O-CO-R^4$ to a hydroxyl group of a compound in which, of the compounds obtained by the above methods (1) and (2), a substituted phenyl group represented by $Ar^2$ is substituted by at least one hydroxyl group, according to a standard method in the art for alkylating or acylating a hydroxyl group.

(5) A compound of formula (I) in which A and B together represent an oxo group can be also produced by first obtaining an alcohol compound according to the above methods (1), (3) or (4) in which A in formula (I) is a hydrogen atom and B is a hydroxyl group, and then oxidizing this compound using an oxidizing agent such as pyridinium dichromate (PDC).

(6) A compound of formula (I) in which A and B together represent a group represented by the following formula:

$$=N-Y$$

wherein Y represents a di-($C_{1-6}$ alkyl) amino group, a hydroxyl group, an aralkyloxy group, or a $C_{1-6}$ alkoxy group can also be produced by first obtaining, by the above method (1), (2), (4) or (5), a compound of formula (I) in which A and B together represent an oxo group, and then, according to a standard method in the art, condensing this compound with amines represented by the following formula:

$$H_2N-Y$$

wherein Y has the aforesaid meaning.

(7) A compound of formula (I) in which A and B together represent a group represented by the following formula:

$$-Z^1-M-Z^2-$$

wherein $Z^1$ and $Z^2$ are the same or different from each other, and each independently represents an oxygen atom,

a sulfur atom, or an imino group optionally substituted with a $C_{1-6}$ alkyl group; and M represents an alkylene group having 2 to 4 chain members or a 1,2-phenylene group
can also be produced by condensing, according to a standard method in the art, a compound of formula (I) in which A and B together represent an oxo group with a bifunctional compound represented by the following formula:

$$H\text{-}Z^1\text{-}M\text{-}Z^2\text{-}H$$

wherein $Z^1$, $Z^2$, and M have the aforesaid meaning.

For example, by subjecting a compound of formula (I) in which A and B together represent an oxo group to treatment with ethylene glycol in benzene in the presence of p-toluenesulfonic acid, it is possible to produce a ketal of formula (I) in which A and B together represent an ethylenedioxy group.

Further, by subjecting a compound of formula (I) in which A and B together represent an oxo group to treatment with 1,2-ethanedithiol in chloroform in the presence of a boron trifluoride-diethyl ether complex, it is possible to produce a thioketal of formula (I) in which A and B together represent an ethylenedithio group.

(8) A compound of formula (I) in which A is a hydroxyl group and B is a 1-$C_{1-6}$-alkyl-imidazol-2-yl group can be produced by treating a compound of formula (I) in which A and B together represent an oxo group with 1-$C_{1-6}$-alkyl-imidazole according to a standard method in the art.

Purification of a product may be conducted according to a commonly applied technique such as, for example, column chromatography employing a silica gel or the like as a carrier, and a recrystallization method using ethyl acetate, acetone, hexane, methanol, ethanol, chloroform, dimethyl sulfoxide, water or the like. Examples of an eluting solvent for column chromatography include chloroform, methanol, acetone, hexane, dichloromethane, ethyl acetate and mixed solvents thereof.

The phosphodiesterase IV inhibitor of the present invention comprises as an effective ingredient a compound represented by the above formula (I) or a pharmaceutically acceptable salt thereof (hereunder, referred to as "pyridylacrylamide derivative (I)"), and is useful as a preventive or therapeutic agent for respiratory diseases such as bronchial asthma and chronic bronchitis; diseases relating to nervous function disorder such as learning, memory and recognition disorders relating to Alzheimer's disease and Parkinson's disease; diseases relating to mental function disorder such as manic-depressive psychosis and schizophrenia; inflammatory diseases such as atopic dermatitis and conjunctivitis; systemic or local articular diseases such as gonarthrosis and rheumatoid arthritis; phosphodiesterase IV-involving diseases such as rheumatoid arthritis, septicemia and Crohn's disease.

Hereunder, a dosage and method of formulating the phosphodiesterase IV inhibitor of the present invention containing the pyridylacrylamide derivative (I) is described.

The pyridylacrylamide derivative (I) can be administered to animal or human as it is or together with a commonly used pharmaceutically prepared carrier. A dosage form is not particularly limited and can be appropriately selected for used according to a need, and examples thereof include an oral agent such as a tablet, capsule, granule, fine granule, or powder, and a parenteral agent such as an injection or suppository.

Although a dosage as an oral agent required to exert a desired effect will differ according to the age, body weight and condition of a patient, under normal conditions a suitable amount of pyridylacrylamide derivative (I) for administration to an adult in one day is from 0.1 mg and 2 g to be divided over several administrations.

An oral agent can be prepared according to a standard method in the art using, for example, starch, lactose, sucrose, mannitol, carboxymethylcellulose, corn starch, inorganic salt, and the like.

In addition to the excipients as described above, this type of formulation can also contain a binder, disintegrator, surfactant, lubricant, enhancer for the fluidity, flavoring agent, colorant, perfume and the like. Respective examples thereof are described below.

[Binders]

Examples of a binder include starch, dextrin, powdered acacia, gelatin, hydroxypropyl starch, methylcellulose, carboxymethylcellulose sodium, hydroxypropylcellulose, crystalline cellulose, ethylcellulose, polyvinylpyrrolidone and Macrogol.

[Disintegrators]

Examples of a disintegrator include starch, hydroxypropyl starch, carboxymethylcellulose sodium, carboxymethylcellulose calcium, carboxymethylcellulose and low-substituted hydroxypropylcellulose.

[Surfactants]

Examples of a surfactant include sodium lauryl sulfate, soybean lecithin, sucrose esters of fatty acid and Polysorbate 80.

[Lubricants]

Examples of a lubricant include talc, waxes, hydrogenated vegetable oil, sucrose esters of fatty acid, magnesium stearate, calcium stearate, aluminum stearate and polyethylene glycol.

[Enhancers for the fluidity]

Examples of an enhancer for the fluidity include light anhydrous silicic acid, dried aluminum hydroxide gel, synthetic aluminum silicate and magnesium silicate.

The pyridylacrylamide derivative (I) can also be administered as a suspension, emulsion, syrup or elixir, and these various dosage forms may include a corrigent and colorant.

Although a dosage as a parenteral agent required to exert a desired effect will differ according to the age, body weight and condition of a patient, under normal conditions a suitable amount of pyridylacrylamide derivative (I) for administration to an adult is an intravenous injection, intravenous drip infusion, subcutaneous injection, or intramuscular injection of 0.01 to 600 mg per day.

The parenteral agent can be prepared according to a standard method in the art, and in general, distilled water for injection, physiological saline, aqueous glucose solution, vegetable oil for injection, sesame oil, peanut oil, soybean oil, corn oil, propylene glycol, polyethylene glycol, and the like can be used as a diluent. A disinfectant, antiseptic, or stabilizer may also be added according to a need. From the viewpoint of stability, the parenteral agent may also be refrigerated after filling into a vial or the like, moisture removed by a common lyophillization technique, and a solution re-prepared from the lyophilized product immediately prior to use. An isotonicity, stabilizer, antiseptic, soothing agent or the like may also be added thereto as required.

Examples of other parenteral agents include paints such as an external liquid preparation or ointment, and a suppository for intrarectal administration. These agents can be produced in accordance with a standard method in the art.

This specification includes part or all of the contents as disclosed in the specification of Japanese Patent Application No. 2002-99491, which is a priority document of the present application.

Best Mode for Carrying Out the Invention

The present invention will be explained further in detail below by the use of preparation examples and an example. However, the following preparation examples and example are not intended to limit the scope of the invention.

Preparation example 1

Synthesis of 2-cyano-N-[4-(methoxymethoxy)phenethyl]-3-(3-pyridyl)-2-propenamide

(compound 1)

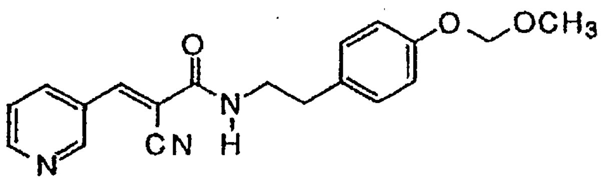

(1)

1.58 g (8.7 mmol) of 4-(methoxymethoxy)phenethylamine and 0.82 g (9.6 mmol) of cyanoacetic acid were dissolved in 10 mL of dimethylformamide, and 1.51 mL (9.6 mmol) of diethyl phosphorocyanidate and 1.34 mL (9.6 mmol) of triethylamine were then added thereto in order while stirring on ice. After stirring for 24 hr at room temperature, aqueous saturated sodium bicarbonate was added to the reaction solution, the solution was extracted with ethyl acetate,

washed with water, and then dried over magnesium sulfate. After removing the solvent by vacuum evaporation, the residue was purified by silica gel column chromatography (chloroform:methanol = 19:1) to obtain 0.97 g (45%) of 2-cyano-N-[4-(methoxymethoxy)phenethyl]acetamide.
Property: solid
$^1$H-NMR ($CDCl_3$)δ: 2.80 (2H, t, J=7Hz), 3.32 (2H, s), 3.48 (3H, s), 3.53 (2H, td, J=7, 6Hz), 5.16 (2H, s), 6.11 (1H, br), 7.00 (2H, br d, J=9Hz), 7.12 (2H, br d, J=9Hz)

Next, 10 mL of ethanol, 0.62 g (5.8 mmol) of 3-pyridinecarbaldehyde and 1 drop of piperidine were added to 0.96 g (3.87 mmol) of the obtained 2-cyano- N-[4-(methoxymethoxy)phenethyl]acetamide, and the resulting mixture was heated under reflux for 19 hours. The reaction solution was concentrated under vacuum, and after purifying the residue by silica gel column chromatography (chloroform:methanol = 19:1) the product was recrystallized to obtain 0.83 g (64%) of the title compound.
Property: mp 105-106°C (ethyl acetate-hexane)
$^1$H-NMR ($CDCl_3$)δ: 2.87 (2H, t, J=7Hz), 3.48 (3H, s), 3.66 (2H, td, J=7, 6Hz), 5.17 (2H, s), 6.42 (1H, br), 7.02 (2H, br d, J=9Hz), 7.16 (2H, br d, J=9Hz), 7.45 (1H, dd, J=8, 5Hz), 8.33 (1H, s), 8.41 (1H, ddd, J=8, 2, 2Hz), 8.73 (1H, dd, J=5, 2Hz), 8.94 (1H, d, J=2Hz)

Preparation example 2

Compound 2 was obtained by a method according to preparation example 1.

Compound 2

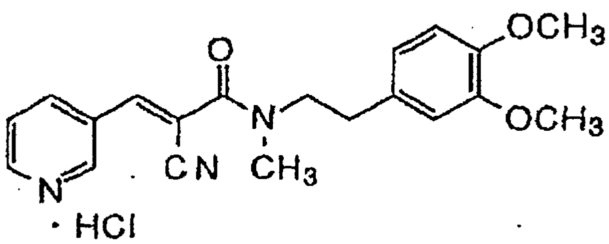

(2)

Property: mp 115-120°C (ethanol-ether)
$^1$H-NMR (DMSO-$d_6$, 100°C) δ: 2.87 (2H, t, J=7Hz), 3.07 (3H, s), 3.71 (2H, t, J=7Hz), 3.73 (6H, s), 6.73-6.89 (3H, m), 7.52 (1H, s), 7.60 (1H, dd, J=8, 5Hz), 8.29 (1H, d, J=8Hz), 8.71 (1H, d, J=5Hz), 8.92 (1H, d, J=2Hz)

Preparation example 3

Synthesis of (E)-N-methyl-3-(3-pyridyl)-N-(3,4,5-trimethoxyphenethyl)-2-propenamide hydrochloride (compound 9)

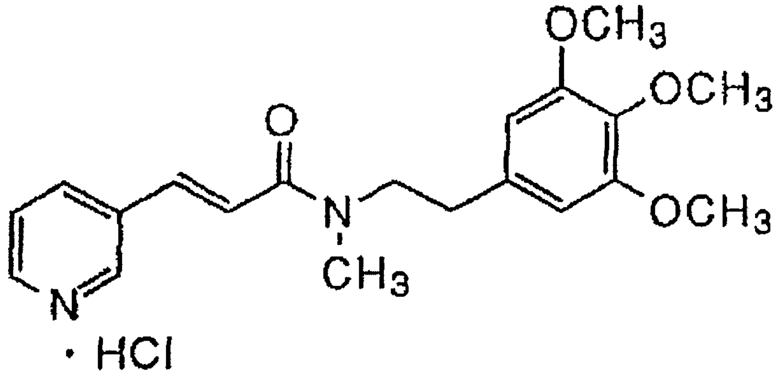

(9)

A mixture of 9.80 g (50 mmol) of 3,4,5-trimethoxybenzaldehyde, 18 mL of nitromethane, 4.11 g of ammonium acetate and 38 mL of acetic acid was heated under reflux for 2 hours. After concentrating the reaction solution under vacuum, 10% aqueous sodium hydroxide was added to the residue, and the residue was then extracted using dichloromethane, washed with water, and then dried over anhydrous magnesium sulfate. After removing the solvent by vacuum evaporation, the residue was purified by silica gel column chromatography (dichloromethane) and recrystallized to obtain 4.96 g (41%) of trans-3,4,5-trimethoxy-β-nitrostyrene.

Property: mp 116-118°C (ethanol)

$^1$H-NMR ($CDCl_3$)δ: 3.91 (6H, s), 3.92 (3H, s), 6.77 (2H, s), 7.54 (1H, d, J=13.6Hz), 7.94 (1H, d, J= 13.6Hz)

A 20 mL tetrahydrofuran solution containing 4.78g (20 mmol) of trans-3,4,5-trimethoxy-β-nitrostyrene was added dropwise into a 20 mL tetrahydrofuran suspension containing 1.52 g of lithium aluminum hydride while stirring on ice. After stirring at room temperature for 3 hrs, 1.5 mL of water, 1.5 mL of 15% aqueous sodium hydroxide and 4.5 mL of water were added dropwise in that order to the reaction mixture while stirring on ice. A small amount of potassium carbonate was then added, and after stirring the mixture for several minutes, the inorganic salts were filtered off and washed with tetrahydrofuran. The filtrate was then concentrated under vacuum. The residue was dissolved in 2N hydrochloric acid and washed with dichloromethane. The aqueous layer was then made basic using sodium hydroxide, and the released oil material was extracted with dichloromethane. After washing, the extracted product was dried over potassium carbonate and the solvent was removed by vacuum evaporation to obtain 3,4,5-trimethoxyphenethylamine as crude oil.

A 30 mL tetrahydrofuran solution of this 3,4,5-trimethoxyphenethylamine crude oil was added at room temperature to a mixed acid anhydride of acetic acid and formic acid (synthesized by adding 6.2 mL of 98% formic acid to 12.5 mL of acetic anhydride while ice-cooling, and allowing the mixture to react for 3 hrs at 60°C) and stirred for 17 hrs. The reaction solution was concentrated under vacuum, and 40 mL of tetrahydrofuran and 12 mL of borane-methyl sulfide complex was then added to the residue while stirring on ice. The resulting mixture was heated under reflux for 17 hrs. After cooling the reaction solution, methanol was added thereto to terminate the reaction, and the solution was concentrated under vacuum. After adding a hydrogen chloride-methanol solution to the residue and heating under reflux for 3 hrs, the solvent was removed by vacuum evaporation. The residue was then dissolved in 2N hydrochloric acid and washed with dichloromethane. An aqueous layer was made basic using sodium hydroxide and the released oil material was extracted using dichloromethane. After washing, the extracted product was dried over potassium carbonate and the solvent was removed by vacuum evaporation, to yield 1.61 g of N-methyl-3,4,5-trimethoxyphenethylamine as colorless oil.

$^1$H-NMR ($CDCl_3$)δ: 2.47 (3H, s), 2.68-2.91 (4H, m), 3.82 (3H, s), 3.86 (6H, s), 6.41 (2H, s)

1.60 g (7.11 mmol) of N-methyl-3,4,5-trimethoxyphenethylamine and 1.17 g of trans-3-(3-pyridyl) acrylic acid were dissolved in 8 mL of dimethylformamide, and 1.3 mL of diethyl phosphorocyanidate and 2.2 ml, of triethylamine were added in that order while stirring on ice. The resulting mixture was then stirred for I hr at room temperature. Aqueous saturated sodium bicarbonate was added to the reaction solution, the solution was extracted with dichloromethane, washed with water, and then dried over potassium carbonate. Following vacuum evaporation of the solvent, the residue was purified by silica gel column chromatography (ethyl acetate: hexane = 10:1) to obtain 2.37 g (94%) of (E)-N-methyl-3-(3-pyridyl)-N-(3,4,5-trimethoxyphenethyl)-2-propenamide as amorphous product. Thereafter, hydrochloric acid-methanol was added to 1.80 g of this product to form a hydrochloride, which was then recrystallized with a mixed solvent of ethyl acetate and methanol to obtain 1.59 g (57%) of the title compound.

Property: mp 164-171°C (ethyl acetate-methanol)

$^1$H-NMR (DMSO-$d_6$, 150°C) δ: 3.04 (2H, t, J=7.1Hz), 3.25 (3H, s), 3.87 (3H, s), 3.94 (2H, t, J=7.1Hz), 3.99 (6H, s),

6.75 (2H, s), 7.24 (1H, d, J=15.6Hz), 7.59 (1H, d, J=15.6Hz), 7.67-7.71 (1H, m), 8.24-8.28 (1H, m), 8.76-8.78 (1H, m), 8.99 (1H, br s)

Preparation examples 4 to 40

The following compounds were obtained by a method in accordance with preparation example 3.

Preparation example 4

Compound 10

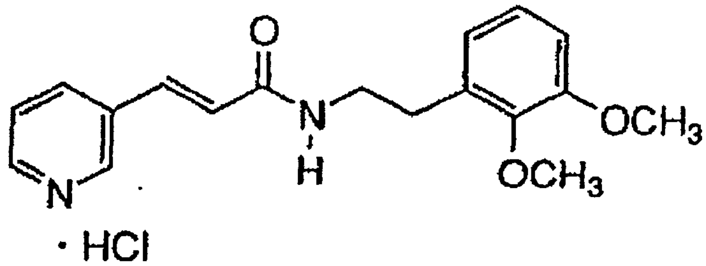

(10)

Property: mp 150-155°C (ethanol)
$^1$H-NMR (DMSO-$d_6$)δ: 2.78 (2H, t, J=7.4Hz), 3.35-3.45 (2H, m), 3.74 (3H, s), 3.79 (3H, s), 6.77-6.82 (1H, m), 6.88-6.94 (1H, m), 6.92 (1H, d, J=15.9Hz), 6.96-7.04 (1H, m), 7.58 (1H, d, J=15.9Hz), 7.97-8.05 (1H, m), 8.49 (1H, t, J=5.7Hz), 8.62-8.67 (1H, m), 8.82-8.86 (1H, m), 9.09 (1H, br s)

Preparation example 5

Compound 11

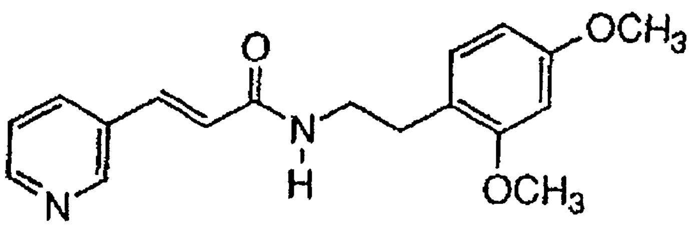

(11)

Property: mp 135.5-136.5°C (ethyl acetate-hexane)
$^1$H-NMR (DMSO-$d_6$)δ: 2.68 (2H, t, J=7.7Hz), 3.29-3.38 (2H, m), 3.73 (3H, s), 3.78 (3H, s), 6.45 (1H, dd, J=8.2, 2.3Hz), 6.54 (1H, d, J=2.3Hz), 6.71 (1H, d, J=15.9Hz), 7.03 (1H, d, J=8.2Hz), 7.41-7.49 (1H, m), 7.45 (1H, d, J=15.9Hz), 7.94-8.00 (1H, m), 8.19 (1H, t, J=5.6Hz), 8.53-8.56 (1H, m), 8.74 (1H, br s)

Preparation example 6

Compound 12

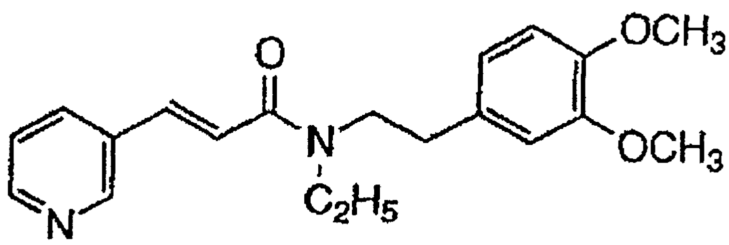

(12)

Property: oil

$^1$H-NMR (DMSO-$d_6$, 100°C) δ: 1.13 (3H, t, J=7.3Hz), 2.80 (2H, t, J=7.3Hz), 3.45 (2H, q, J=7.3Hz), 3.63 (2H, t, J=7.3Hz), 3.70 (3H, s), 3.74 (3H, s), 6.75 (1H, dd, J=8.3, 2.0Hz), 6.82 (1H, d, J=2.0Hz), 6.83 (1H, d, J=8.3Hz), 6.96 (1H, d, J=15.6Hz), 7.32-7.36 (1H, m), 7.38 (1H, d, J=15.6Hz), 7.89-7.92 (1H, m), 8.48-8.51 (1H, m), 8.71 (1H, br s)

Preparation example 7

Compound 13

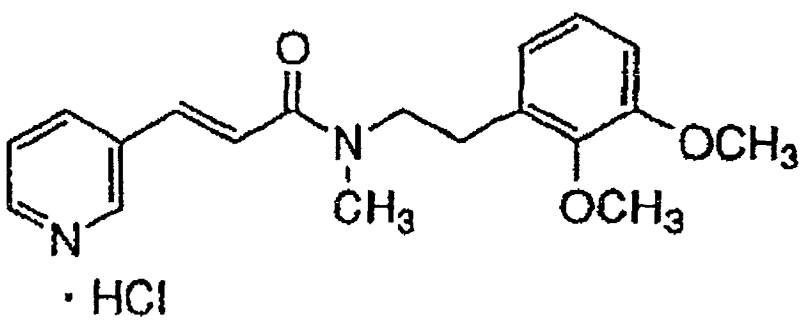

(13)

Property: mp 160-163°C (ethanol)

$^1$H-NMR (DMSO-$d_6$, 120°C) δ: 2.85 (2H, t, J=7.2Hz), 3.01 (3H, s), 3.61-3.69 (2H, m), 3.75 (3H, s), 3.77 (3H, s), 6.78 (1H, dd, J=7.1, 2.0Hz), 6.85 (1H, dd, J=8.0, 2.0Hz), 6.93 (1H, dd, J=8.0, 7.1Hz), 7.10 (1H, d, J=15.6Hz), 7.39 (1H, d, J=15.6Hz), 7.54-7.61 (1H, m), 8.19 (1H, d, J=7.4Hz), 8.59 (1H, d, J=4.8Hz), 8.83 (1H, s)

Preparation example 8

Compound 14

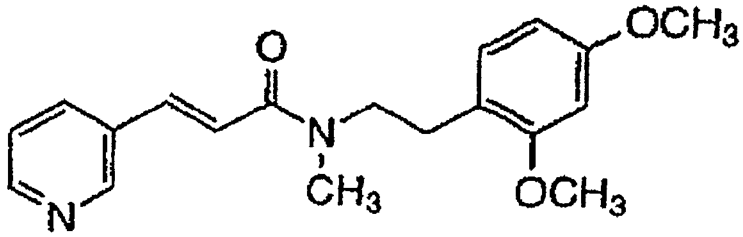

(14)

Property: mp 84-88°C (ethyl acetate-hexane)
$^1$H-NMR (DMSO-$d_6$, 100°C) δ: 2.76 (2H, t, J=7.3Hz), 2.96 (3H, s), 3.59 (2H, t, J=7.3Hz), 3.68 (3H, s), 3.76(3H, s), 6.39-6.47 (2H, m), 6.93-7.05 (2H, m), 7.28-7.41 (2H, m), 7.88-7.98 (1H, m), 8.50-8.52 (1H, m), 8.72 (1H, br s)

Preparation example 9

Compound 15

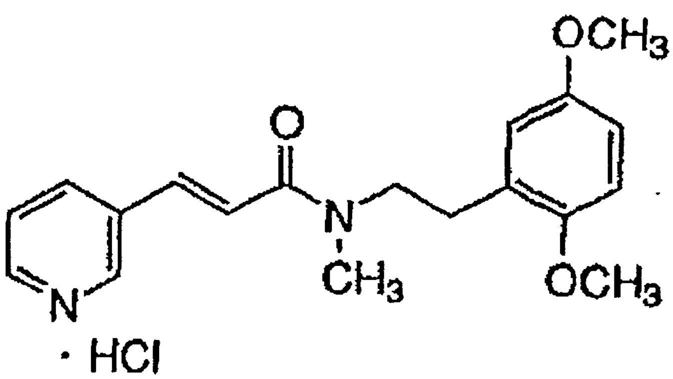

(15)

Property: mp 153-156°C (ethanol)
$^1$H-NMR (DMSO-$d_6$, 120°C) δ: 2.82 (2H, t, J=7.1Hz), 2.99 (3H, s), 3.65 (2H, t, J=7.1Hz), 3.66 (3H, s), 3.75 (3H, s), 6.69 (1H, dd, J=8.7, 2.9Hz), 6.75 (1H, d, J=2.9Hz), 6.83 (1H, d, J=8.7Hz), 7.08 (1H, d, J=15.6Hz), 7.37 (1H, d, J=15.6Hz), 7.59 (1H, dd, J=7.9,5.1Hz), 8.19 (1H, d, J=7.9Hz), 8.59 (1H, d, J=5.1Hz), 8.83 (1H, s)

Preparation example 10

Compound 16

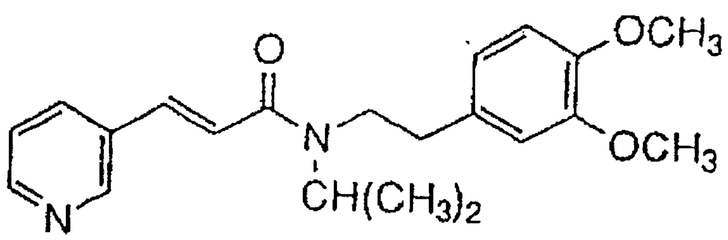

(16)

Property: oil

$^1$H-NMR (DMSO-$d_6$, 100°C) δ: 1.20 (6H, d, J=6.7Hz), 2.80 (2H, t, J=7.3Hz), 3.52 (2H, t, J=7.3Hz), 3.70 (3H, s), 3.75 (3H, s), 4.45 (1H, septet, J=6.7Hz), 6.74-6.87 (3H, m), 7.07 (1H, d, J=15.6Hz), 7.33-7.40 (1H, m), 7.42 (1H, d, J=15.6Hz), 7.95-7.99 (1H, m), 8.49-8.53 (1H, m), 8.76 (1H, m)

Preparation example 11

Compound 17

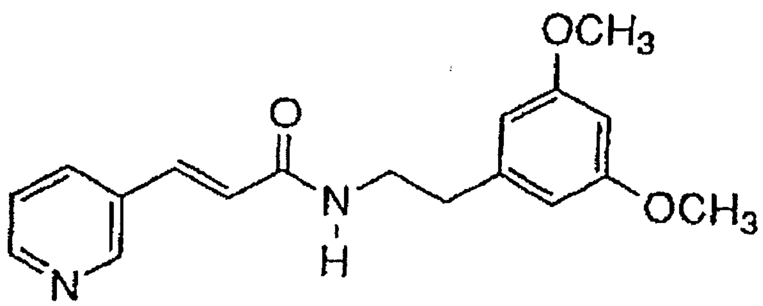

(17)

Property: oil

$^1$H-NMR ($CDCl_3$)δ: 2.84 (2H, t, J=6.9Hz), 3.61-3.71 (2H, m), 3.78 (6H, s), 6.13 (1H, br s), 6.34-6.39 (3H, m), 6.44 (1H, d, J=15.7Hz), 7.28 (1H, dd, J=7.9, 4.8Hz), 7.60 (1H, d, J=15.7Hz), 7.75 (1H, d, J=7.9Hz), 8.53 (1H, d, J=4.8Hz), 8.68 (1H, br s)

Preparation example 12

Compound 18

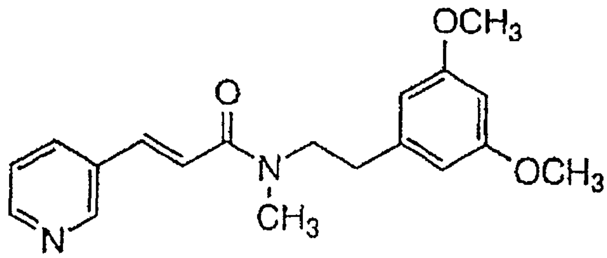

(18)

Property: oil
$^1$H-NMR (DMSO-$d_6$, 100°C) δ: 2.79 (2H, t, J=7.3Hz), 2.97 (3H, s), 3.65-3.73 (2H, m), 3.69 (6H, s), 6.29 (1H, d, J=2.4Hz), 6.40 (2H, d, J=2.4Hz), 7.04 (1H, br), 7.29-7.40 (2H, m), 7.92-8.01 (1H, m), 8.50-8.52 (1H, m), 8.74 (1H, br s)

Preparation example 13

Compound 19

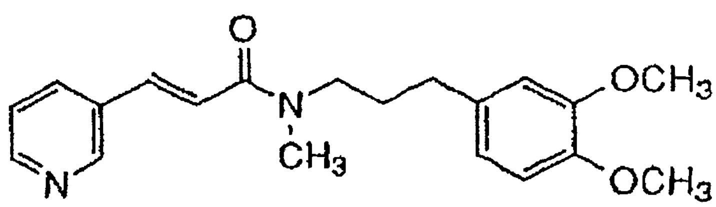

(19)

Property: oil
$^1$H-NMR (DMSO-$d_6$, 100°C) δ: 1.75-1.97 (2H, m), 2.50-2.55 (2H, m), 3.02 (3H, s), 3.46 (2H, t, J=7.2Hz), 3.71 (3H, s), 3.73 (3H, s), 6.70-6.85 (3H, m), 7.09 (1H, d, J=15.7Hz), 7.34-7.38 (1H, m), 7.44 (1H, d, J=15.7Hz), 7.95-7.99 (1H, m), 8.50-8.52 (1H, m), 8.76 (1H, d, J=2.0Hz)

Preparation example 14

Compound 20

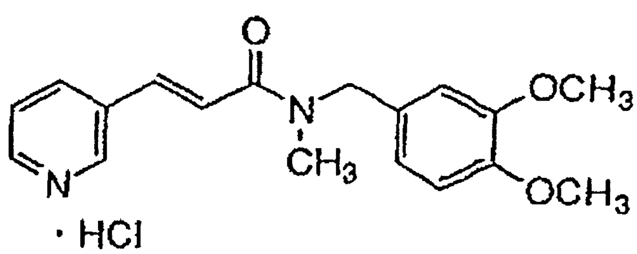

(20)

Property: amorphous
$^1$H-NMR (DMSO-$d_6$, 100°C) δ: 3.01 (3H, s), 3.74 (6H, s), 4.61 (2H, s), 6.65-6.94 (3H, m), 7.38 (1H, d, J=15.6Hz), 7.57 (1H, d, J=15.6Hz), 7.61-7.66 (1H, m), 8.33-8.37 (1H, m), 8.60-8.63 (1H, m), 8.95 (1H, br s)

Preparation example 15

Compound 21

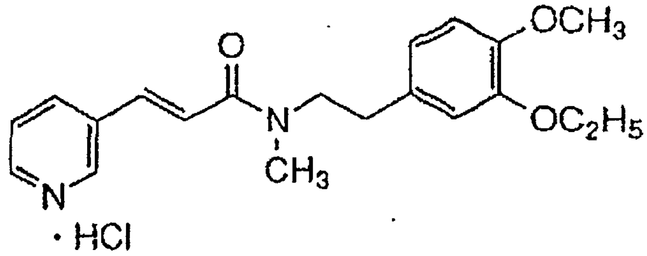

(21)

Property: mp 182-186°C (ether-methanol)
$^1$H-NMR (DMSO-$d_6$, 100°C) δ: 1.27 (3H, t, J=6.9Hz), 2.77 (2H, t, J=6.9Hz), 2.99 (3H, s), 3.67-3.73 (5H, m), 3.94-4.03 (2H, m), 6.71-6.84 (3H, m), 7.04-7.14 (1H, m), 7.33-7.40 (1H, m), 7.56-7.66 (1H, m), 8.23-8.27 (1H, m), 8.65-8.67 (1H, m), 8.92 (1H, br s)

Preparation example 16

Compound 22

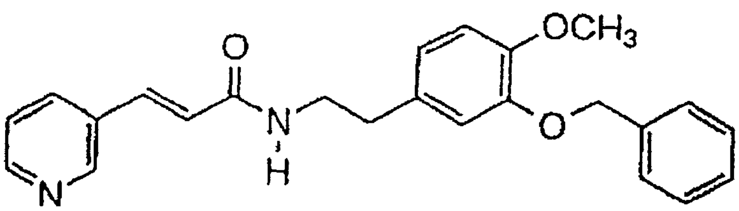

(22)

Property: mp 152-154°C (dichloromethane-hexane)
$^1$H-NMR ($CDCl_3$)δ: 2.78 (2H, t, J=6.7Hz), 3.54-3.64 (2H, m), 3.87 (3H, s), 5.14 (2H, s), 5.64 (1H, m), 6.32 (1H, d, J=15.7Hz), 6.75-6.88 (3H, m), 7.22-7.45 (6H, m), 7.59 (1H, d, J=15.7Hz), 7.73-7.79 (1H, m), 8.56 (1H, dd, J=4.8, 1.7Hz), 8.70-8.71(1H, m)

Preparation example 17

Compound 29

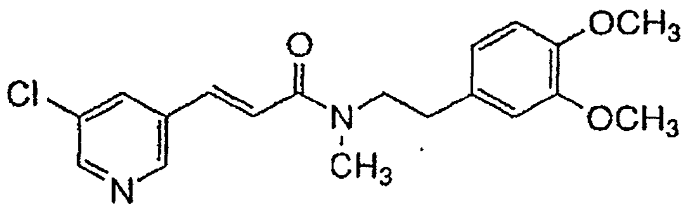

(29)

Property: mp 138-140°C (ethyl acetate-hexane)
$^{1}$H-NMR (DMSO-$d_6$, 150°C) δ: 2.79 (2H, t, J=7.1Hz), 2.99 (3H, s), 3.67 (2H, t, J=7.1 Hz), 3.69 (3H, s), 3.73 (3H, s), 6.70-6.84 (3H, m), 7.02 (1H, d, J=15.6Hz), 7.29 (1H, d, J=15.6Hz), 8.02 (1H, br s), 8.48 (1H, br s), 8.62 (1H, br s)

Preparation example 18

Compound 30

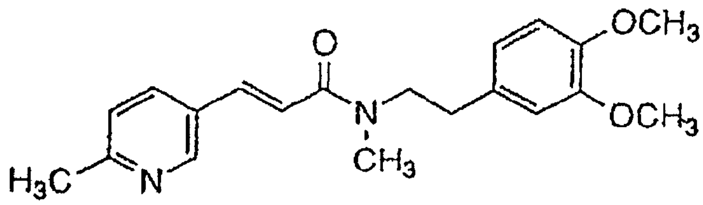

(30)

Property: mp 124.5-125.5°C (ethyl acetate-hexane)
$^{1}$H-NMR (DMSO-$d_6$, 150°C) δ: 2.47 (3H, s), 2.79 (2H, t, J=7.2Hz), 2.99 (3H, s), 3.66 (2H, t, J=7.2Hz), 3.70 (3H, s), 3.74 (3H, s), 6.71-6.93 (4H, m), 7.19 (1H, d, J=8.1Hz), 7.31 (1H, d, J=15.8Hz), 7.77 (1H, d, J=8.1Hz), 8.55 (1H, br s)

Preparation example 19

Compound 31

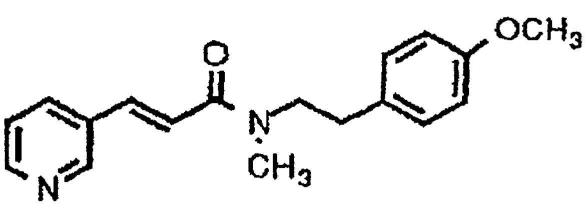

(31)

Property: mp 81-84°C (dichloromethane-hexane)
$^{1}$H-NMR (DMSO-$d_6$, 150°C) δ: 2.80 (2H, t, J=7.2Hz), 2.98 (3H, s), 3.64 (2H, t, J=7.2Hz), 3.69 (3H, s), 6.81(2H, d, J=8.4Hz), 6.95 (1H, d, J=15.6Hz), 7.12 (2H, d, J=8.4Hz), 7.33 (1H, d, J=15.6Hz), 7.29-7.37 (1H, m), 7.88-7.93 (1H, m), 8.48-8.50 (1H, m), 8.69-8.70 (1H, m)

Preparation example 20

Compound 32

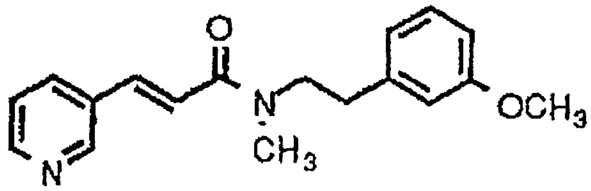

(32)

Property: mp 81-84°C (ethyl acetate-hexane)
$^1$H-NMR (DMSO-$d_6$, 100°C) δ: 2.83 (2H, t, J=7.3Hz), 2.95 (3H, s), 3.66-3.73 (2H, m), 3.71 (3H, s), 6.70-6.82 (3H, m), 6.98-7.20 (2H, m), 7.32-7.40 (2H, m), 7.94-7.98 (1H, m), 8.49-8.52 (1H, m), 8.73-8.74 (1H, m)

Preparation example 21

Compound 33

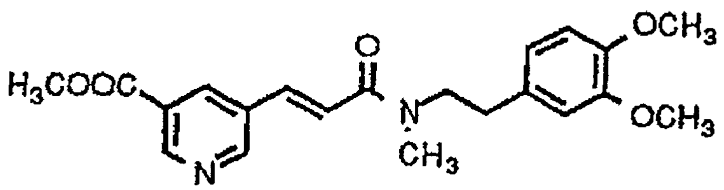

(33)

Property: mp 165-167°C (ethyl acetate-hexane)
$^1$H-NMR (DMSO-$d_6$, 150°C) δ: 2.77-2.83 (2H, m), 3.00 (3H, s), 3.64-3.72 (2H, m), 3.68 (3H, s), 3.73 (3H, s), 3.92 (3H, s), 6.71-6.84 (3H, m), 7.03 (1H, d, J=15.9Hz), 7.37 (1H, d, J=15.9Hz), 8.34 (1H, br s), 8.89 (1H, br s), 8.97(1H, br s)

Preparation example 22

Compound 34

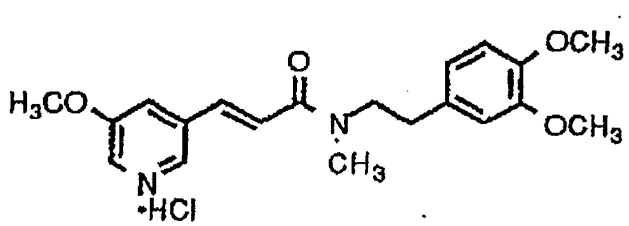

(34)

Property: solid
$^1$H-NMR (DMSO-$d_6$, 150°C) δ: 2.79 (2H, t, J=7.2Hz), 2.99 (3H, s), 3.67 (2H, t, J=7.2Hz), 3.69 (3H, s), 3.73 (3H, s), 3.88 (3H, s), 6.71-6.84 (3H, m), 6.98 (1H, d, J=15.6Hz), 7.32 (1H, d, J=15.6Hz), 7.53 (1H, br s), 8.23-8.24 (1H, m), 8.32 (1H, br s)

Preparation example 23

Compound 35

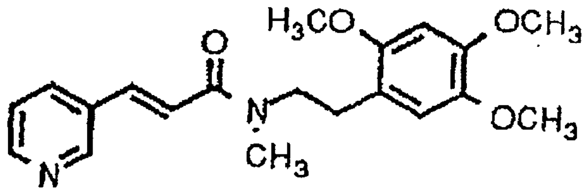

(35)

Property: mp 71-74°C (ethyl acetate-hexane)
$^1$H-NMR (DMSO-$d_6$, 100°C) δ: 2.77 (2H, t, J=7.0Hz), 2.98 (3H, s), 3.61 (2H, t, J=7.0Hz), 3.68 (3H, s), 3.72 (3H, s), 3.74 (3H, s), 6.58 (1H, s), 6.74 (1H, s), 6.93 (1H, d, J=15.6Hz), 7.28-7.36 (2H, m), 7.85-7.89 (1H, m), 8.48-8.50 (1H, m), 8.68 (1H, br s)

Preparation example 24

Compound 36

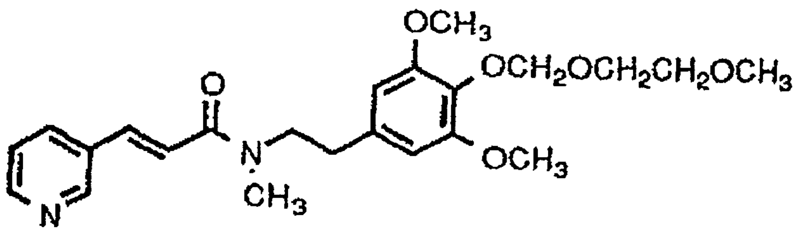

(36)

Property: amorphous
$^1$H-NMR (DMSO-$d_6$, 150°C) δ: 2.78-2.85 (2H, m), 3.01 (3H, s), 3.25 (3H, s), 3.44-3.49 (2H, m), 3.63-3.81 (4H, m), 3.74 (6H, s), 4.96 (2H, s), 6.48-6.53 (2H, m), 6.94-7.02 (1H, m), 7.30-7.39 (2H, m), 7.87-7.91 (1H, m), 8.48-8.50 (1H, m), 8.70 (1H, br s)

Preparation example 25

Compound 37

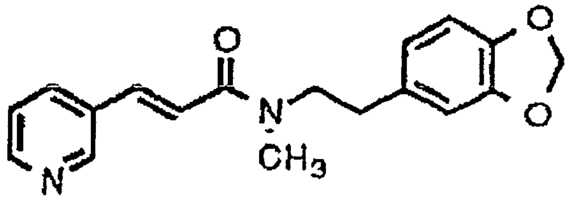

(37)

Property: mp 93-95°C (ethyl acetate-hexane)
$^1$H-NMR (DMSO-$d_6$, 150°C) δ: 2.78 (2H, t, J=7.2Hz), 2.99 (3H, s), 3.64(2H, t, J=7.2Hz), 5.85 (2H, s), 6.63-6.76 (3H, m), 6.97 (1H, d, J=15.6Hz), 7.30-7.37 (2H, m), 7.89-7.93 (1H, m), 8.48-8.50 (1H, m), 8.70 (1H, br s)

Preparation example 26

Compound 39

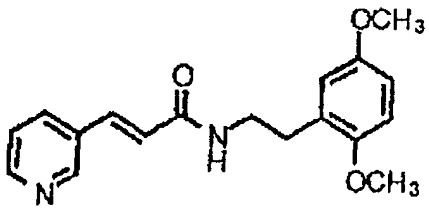

(39)

Property: mp 105-107°C (ethyl acetate)
$^1$H-NMR (DMSO-$d_6$)δ: 2.77 (2H, t, J=7.2Hz), 3.36-3.47 (2H, m), 3.69 (3H, s), 3.75 (3H, s), 6.68 (1H, d, J=15.7Hz), 6.69-6.75 (2H, m), 6.84-6.90 (1H, m), 7.34-7.41 (1H, m), 7.42 (1H, d, J=15.7Hz), 7.70-7.86 (1H, br), 7.86-7.92 (1H, m), 8.49-8.52 (1H, m), 8.69-8.70 (1H, m)

Preparation example 27

Compound 40

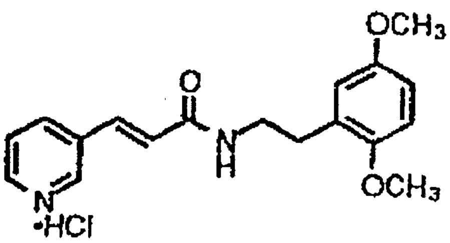

(40)

Property: mp 144-146°C (ethanol)
$^1$H-NMR (DMSO-$d_6$)δ : 2.75 (2H, t, J=7.2Hz), 3.34-3.47 (2H, m), 3.68 (3H, s), 3.74 (3H, s), 6.72-6.78 (2H, m), 6.86-6.92 (1H, m), 6.94 (1H, d, J=15.9Hz), 7.57 (1H, t,J=15.9Hz), 7.95-8.02 (1H, m), 8.45-8.52 (1H, br), 8.61 (1H, d, J=8.1Hz), 8.83 (1H, d, J=5.4Hz), 9.08 (1H, s)

Preparation example 28

Compound 41

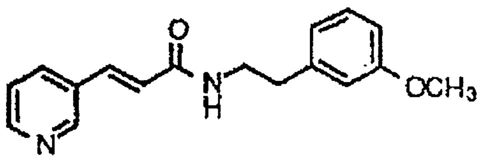

(41)

Property: mp 90-92°C (chloroform-hexane)
$^1$H-NMR ($CDCl_3$)δ : 2.88 (3H, t, J=6.8Hz), 3.64-3.70 (2H, m), 3.80 (3H, s), 5.80-5.90 (1H, br), 6.40 (1H, d, J=15.6Hz), 6.77-6.83 (3H, m), 7.24 (1H, t, J=7.8Hz), 7.27-7.31 (1H, m), 7.61 (1H, d, J=15.6Hz), 7.74-7.78 (1H, m), 8.53-8.56 (1H, m), 8.69-8.71 (1H, m)

Preparation example 29

Compound 42

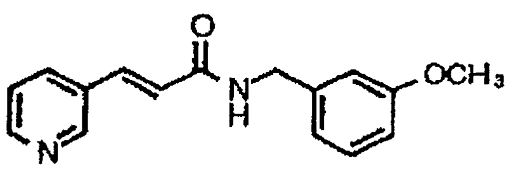

(42)

Property: mp 81-83°C (chloroform-ether)
$^1$H-NMR ($CD_3OD$)δ : 3.77 (3H, s), 4.45-4.49 (2H, m), 6.77 (1H, d, J=15.9Hz), 6.79-6.92 (3H, m), 7.24 (1H, t, J=8.1Hz), 7.43-7.50 (1H, m), 7.59 (1H, d, J=15.9Hz), 8.01-8.08 (1H, m), 8.49-8.52 (1H, m), 8.71 (1H, br s)

Preparation example 30

Compound 43

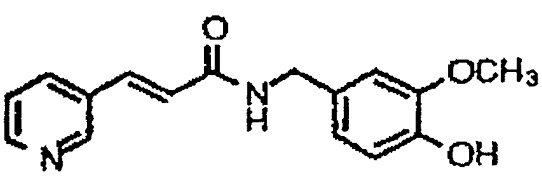

(43)

Property: mp 160-162°C (methanol)
$^1$H-NMR ($CD_3OD$)δ : 3.85 (3H, s), 4.41 (2H, s), 6.72-6.81 (2H, m), 6.75 (1H, d, J=15.9Hz), 6.90-6.92 (1H, m), 7.46 (1H, dd, J=8.0, 4.9Hz), 7.59 (1H, d, J=15.9Hz), 8.00-8.07 (1H, m), 8.50 (1H, dd, J=4.9, 1.5Hz), 8.71 (1H, d, J=2.1Hz)

Preparation example 31

Compound 44

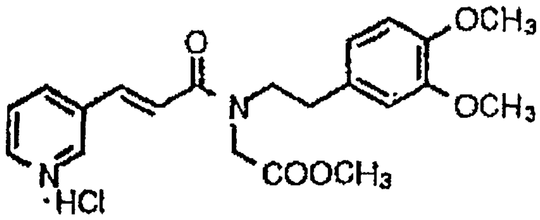

(44)

Property: mp 168-170°C (methanol)
$^1$H-NMR (DMSO-$d_6$, 100°C) δ : 2.80 (2H, t, J=7.0Hz), 3.60-3.80 (11H, m), 4.10-4.30 (2H, m), 6.72-6.86 (3H, m), 7.05 (1H, d, J=15.3Hz), 7.40 (1H, d, J=15.3Hz), 7.63 (1H, dd, J=8.1, 5.1Hz), 8.22-8.27 (1H, m), 8.60-8.64 (1H, m), 8.87 (1H, s)

Preparation example 32

Compound 47

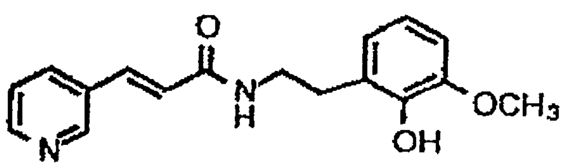

(47)

Property: mp 174-176°C (methanol)
$^1$H-NMR (DMSO-$d_6$)δ: 2.74 (2H, t, J=7.4Hz), 3.30-3.42 (2H, m), 3.78 (3H, s), 6.67-6.86 (3H, m), 6.71 (1H, d, J=15.9Hz), 7.41-7.48 (1H, m), 7.45 (1H, d, J=15.9Hz), 7.94-8.00 (1H, m), 8.21-8.27 (1H, br), 8.53-8.56 (1H, m), 8.56 (1H, s), 8.75 (1H, d, J=2.0Hz)

Preparation example 33

Compound 49

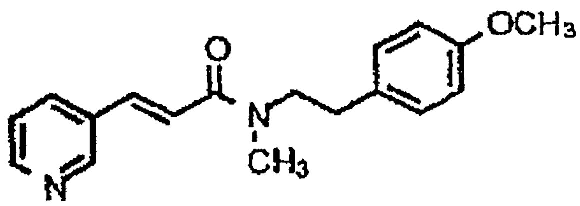

(49)

Property: mp 113-115°C (acetone)

$^1$H-NMR (DMSO-$d_6$, 100°C) δ : 2.75-2.82 (2H, m), 2.98 (3H, s), 3.60-3.68 (2H, m), 3.68 (3H, s), 6.77-6.84 (2H, m), 6.98 (1H, d, J=15.7Hz), 7.09-7.16 (2H, m), 7.30-7.36 (1H, m), 7.34 (1H, d, J=15.7Hz), 7.89-7.96 (1H, m), 8.48-8.52 (1H, m), 8.71 (1H, d, J=2.1Hz)

Preparation example 34

Compound 50

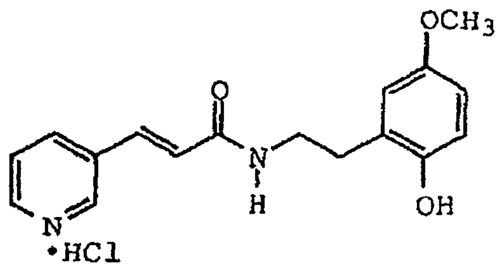

(50)

Property: mp 185-187°C (methanol)
$^1$H-NMR ($CD_3OD$)δ : 2.85 (2H, t, J=7.2Hz), 3.57 (2H, t, J=7.2Hz), 3.70 (3H, s), 6.59-6.73 (3H, m), 6.92 (1H, d, J=15.9Hz), 7.63 (1H, d, J=15.9Hz), 8.06-8.13 (1H, m), 8.80-8.83 (2H, m), 9.06 (1H, s)

Preparation example 35

Compound 51

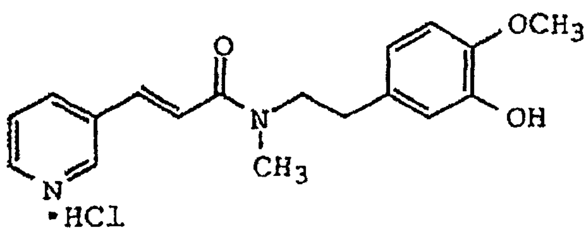

(51)

Property: amorphous
$^1$H-NMR (DMSO-$d_6$, 100°C) δ: 2.75 (2H, t, J=7.5Hz), 3.02 (3H, s), 3.66 (2H, t, J=7.5Hz), 3.73 (3H, s), 6.64 (1H, dd, J=8.1, =2.1Hz), 6.72 (1H, d, J=2.1Hz), 6.82 (1H, d, J=8.1Hz), 6.95-7.17 (1H, m), 7.41 (1H, d, J=15.7Hz), 7.52-7.82 (1H, m), 8.17 (1H, m), 8.59-8.61 (1H, m), 8.85 (1H, m)

Preparation example 36

Compound 54

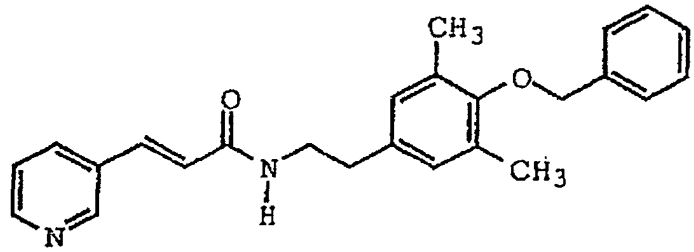

(54)

Property: amorphous
$^1$H-NMR ($CDCl_3$)δ : 2.29 (6H, s), 2.79 (2H, t, J=6.9Hz), 3.58-3.68 (2H, m), 4.80 (2H, s), 5.88 (1H, m), 6.41 (1H, d, J=15.7Hz), 6.89 (2H, s), 7.29-7.51 (6H, m), 7.62 (1H, d, J=15.7Hz), 7.74-7.77 (1H, m), 8.55 (1H, dd, J=4.8,1.5Hz), 8.71 (1H, d, J=1.9Hz)

Preparation example 37

Compound 55

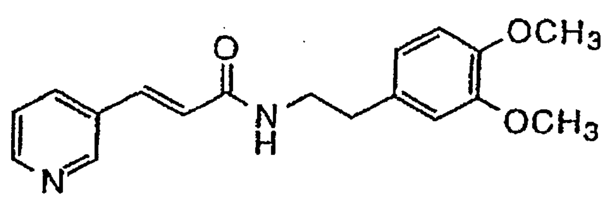

(55)

Property: mp 113-114°C (ethyl acetate-hexane)
$^1$H-NMR ($CDCl_3$)δ: 2.85 (2H, t, J=6.8Hz), 3.66 (2H, td, J=6.8, 6.8Hz), 3.87 (6H, s), 5.73 (1H, br), 6.39 (1H, d, J=15.6Hz), 6.74-6.86 (3H, m), 7.30 (1H, dd, J=7.6, 4.9Hz), 7.62 (1H, d, J=15.6Hz), 7.77 (1H, ddd, J=7.6, 2.2, 1.7Hz), 8.56 (1H, dd, J=4.9, 1.7Hz), 8.72 (1H, d, J=2.2Hz)

Preparation example 38

Compound 61

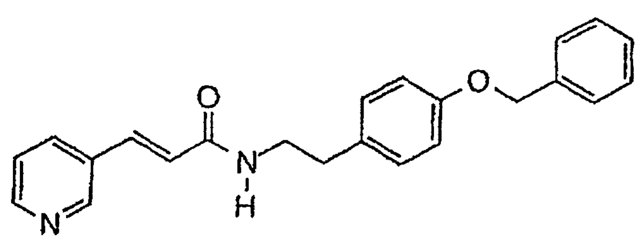

(61)

Property: mp 157-158°C (ethanol-ethyl acetate)
$^1$H-NMR ($CDCl_3$)δ: 2.84 (2H, t, J=7Hz), 3.64 (2H, td, J=7, 6Hz), 5.01 (2H, s), 5.67 (1H, br), 6.38 (1H, d, J=16Hz), 6.94 (2H, d, J=9Hz), 7.14 (2H, d, J=9Hz), 7.29-7.45 (6H, m), 7.61 (1H, d, J=16Hz), 7.77 (1H, d, J=8Hz), 8.56 (1H, dd, J=5, 2Hz), 8.72 (1H, d, J=2Hz)

Preparation example 39

Compound 62

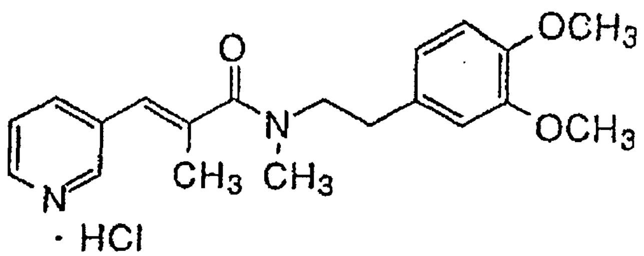

(62)

Property: amorphous
$^1$H-NMR (DMSO-$d_6$, 100°C) δ: 1.89 (3H, d, J=1Hz), 2.80 (2H, t, J=7Hz), 2.95 (3H, s), 3.59 (2H, t, J=7Hz), 3.70 (3H, s), 3.72 (3H, s), 6.26 (1H, br s), 6.72 (1H, dd, J=8, 2Hz), 6.79 (1H, d, J=2Hz), 6.84 (1H, d, J=8Hz), 7.34-7.38 (1H, m), 7.68-7.70 (1H, m), 8.44-8.45 (1H, m), 8.49-8.50 (1H, m)

Preparation example 40

Compound 63

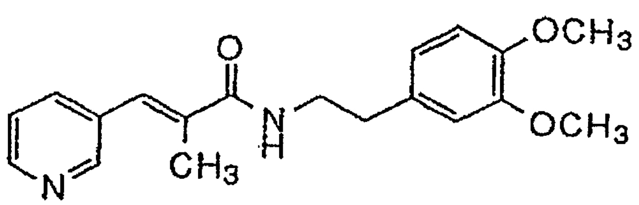

(63)

Property: oil
$^1$H-NMR ($CDCl_3$)δ: 2.05 (3H, d, J=1Hz), 2.86 (2H, t, J=7Hz), 3.63 (2H, td, J=7, 6Hz), 3.87 (6H, s), 6.12 (1H, t, J=6Hz), 6.75-6.86 (3H, m), 7.25-7.34 (2H, m), 7.59-7.63 (1H, m), 8.49-8.54 (2H, m)

Preparation example 41

Compound 66 was obtained by conducting acid hydrolysis of the compound (compound 36) obtained in preparation example 24 by a standard method in the art.

Compound 66

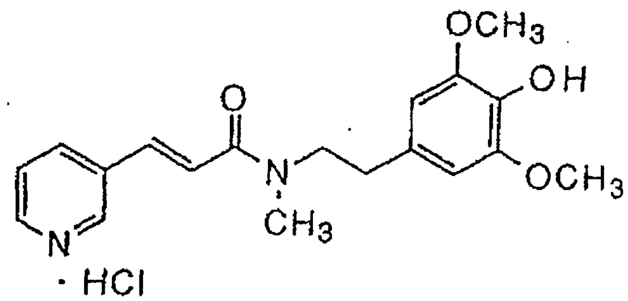

(66)

Property: mp 152-155°C (methanol)
$^1$H-NMR (DMSO-$d_6$, 150°C) δ: 2.75 (2H, t, J=7.1Hz), 2.99 (3H, s), 3.66 (2H, t, J=7.1Hz), 3.73 (6H, s), 6.64 (2H, s), 6.98 (1H, d, J=15.6Hz), 7.33 (1H, d, J=15.6Hz), 7.34-7.40 (1H, m), 7.92-7.96 (1H, m), 8.49-8.51 (1H, m), 8.72 (1H, br s)

Preparation example 42

Synthesis of (Z)-N-(3-methoxyphenethyl)-3-phenyl-3-(3-pyridyl)-2-propenamide (compound 69)

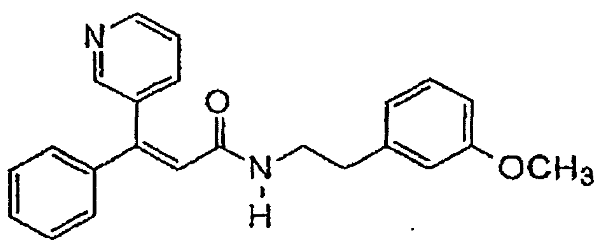

(69)

A mixture containing 528 mg of 60% sodium hydride, 2.20 g of methyl dimethylphosphonoacetate and 100 mL of tetrahydrofuran was stirred at room temperature for 1 hr, then 2.01 g of 3-benzoylpyridine was added while stirring on ice, and the resultant mixture was stirred at room temperature for 18 hours. The reaction mixture was poured onto an ice-bath, extracted with ethyl acetate, washed with water, and then dried over magnesium sulfate. After removing the solvent by vacuum evaporation, 4.40 g of sodium hydroxide, 22 mL water and 22 mL methanol were added to the residue and the mixture was stirred for 5 h at room temperature. The reaction solution was made acidic using hydrogen chloride/methanol, and concentrated under reduced pressure. The precipitated inorganic salts were washed with ethanol-ethyl acetate and filtered off. After concentrating the filtrate, the residue was recrystallized to obtain 1.00 g (35%) of (Z)-3-phenyl-3-(3-pyridyl)-2-propenoic acid hydrochloride as a primary crystal.
$^1$H-NMR ($CD_3OD$)δ: 6.74 (1H, s), 7.35-7.52 (5H, m), 8.09-8.16 (1H, m), 8.43-8.49 (1H, m), 8.73-8.90 (2H, m)

0.40 g (14%) of (E)-3-phenyl-3-(3-pyridyl)-2-propenoic acid hydrochloride was further obtained from the mother liquor as secondary crystals.
$^1$H-NMR ($CD_3OD$)δ: 6.73 (1H, s), 7.27-7.50 (5H, m), 8.05-8.15 (1H, m), 8.49-8.57 (1H, m), 8.78-8.91 (2H, m)

Using 1.00 g of (Z)-3-phenyl-3-(3-pyridyl)-2-propenoic acid hydrochloride and 0.60 g of 3-methoxyphenethylamine as raw material, 1.33 g (98%) of the title compound was obtained by a method in accordance with preparation example 3.
Property: oil
$^1$H-NMR ($CDCl_3$)δ: 2.66 (2H, t, J=7Hz), 3.41-3.51 (2H, m), 3.79 (3H, s), 5.50-5.68 (1H, m), 6.34 (1H, s), 6.64-6.68 (2H, m), 6.73-6.79 (1H, m), 7.18-7.35 (7H, m), 7.52-7.58 (1H, m), 8.45-8.46 (1H, m), 8.57-8.60 (1H, m)

Preparation example 43

Synthesis of (E)-N-(2-methoxyphenethyl)-3-(3-pyridyl)-2-propenamide (compound 72)

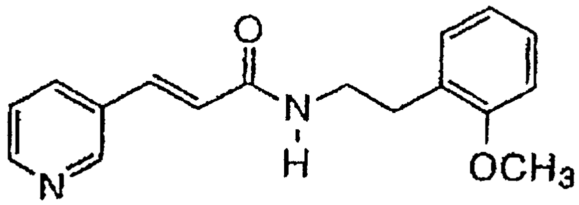

(72)

298 mg of trans-3-(3-pyridyl) acrylic acid and 324 mg of N,N'-carbonyldiimidazole was dissolved in 10 mL of dimethylformamide and stirred at room temperature for 1 h. Subsequently, 302 mg of 2-methoxyphenethylamine was added at room temperature, and the mixture was stirred for 1 h. Water was added to the reaction mixture, the mixture was then extracted with ethyl acetate, washed with water, and dried over magnesium sulfate. The solvent was removed by evaporation to obtain 504 mg (89%) of the title compound. Property: oil
$^1$H-NMR ($CDCl_3$)δ: 2.91 (2H, t, J=7Hz), 3.59-3.68 (2H, m), 3.84 (3H, s), 6.14-6.34 (1H, m), 6.42 (1H, d, J=16Hz), 6.83-7.31 (5H, m), 7.57 (1H, d, J=16Hz), 7.72-7.78 (1H, m), 8.51-8.54 (1H, m), 8.69-8.70 (1H, m)

Preparation examples 44 to 47

Preparation example 44

Compound 73

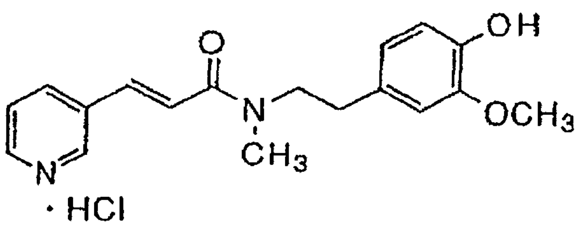

(73)

Property: mp 192-199°C (ethanol-methanol)
$^1$H-NMR (DMSO-$d_6$, 100°C) δ: 2.75 (2H, t, J=7Hz), 2.99 (3H, s), 3.67 (2H, t, J=7Hz), 3.74 (3H, s), 6.60 (1H, dd, J=8, 2Hz), 6.68 (1H, d, J=8Hz), 6.77 (1H, m), 6.89 (1H, br s), 7.17 (1H, d, J=16Hz), 7.38 (1H, d, J=16Hz), 7.73 (1H, dd, J=8, 5Hz), 8.39 (1H, d, J=8Hz), 8.66 (1H, dd, J=5, 1Hz), 8.95 (1H, s)

Preparation example 45

Compound 76

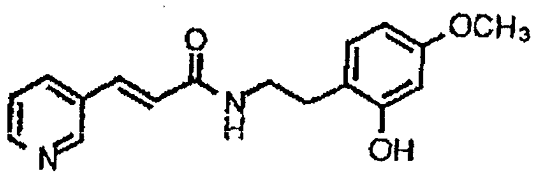

(76)

Property: mp 203-205°C (methanol)
$^1$H-NMR (DMSO-$d_6$)δ: 2.65 (2H, t, J=7.3Hz), 3.27-3.39 (2H, m), 3.66 (3H, s), 6.31 (1H, dd, J=8.2, 2.5Hz), 6.39 (1H, d, J=2.5Hz), 6.72 (1H, d, J=15.8Hz), 6.95 (1H, d, J=8.2Hz), 7.40-7.48 (1H, m), 7.45 (1H, d, J=15.8Hz), 7.94-8.00 (1H, m), 8.18-8.24 (1H, br), 8.53-8.56 (1H, m), 8.75 (1H, d, J=1.9Hz), 9.43 (1H, s)

Preparation example 46

Compound 77

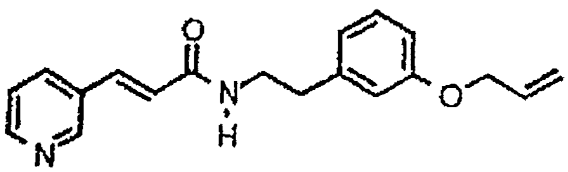

(77)

Property: mp 82.5-84.5°C (ethyl acetate)
$^1$H-NMR ($CDCl_3$)δ: 2.87 (2H, t, J=6.8Hz), 3.62-3.72 (2H, m), 4.51-4.55 (2H, m), 5.28 (1H, dd, J=10.5, 1.5Hz), 5.41 (1H, dd, J=17.3, 1.5Hz), 5.72-5.80 (1H, br), 6.04 (1H, ddd,J=17.3, 10.5, 5.3Hz), 6.39 (1H, d, J=15.7Hz), 6.78-6.84 (3H, m), 7.19-7.33 (2H, m), 7.61 (1H, d, J=15.7Hz), 7.73-7.80 (1H, m), 8.53-8.57 (1H, m), 8.71 (1H, d, J=1.7Hz)

Preparation example 47

Compound 78

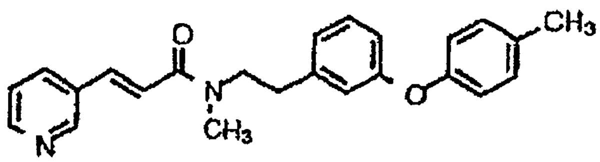

(78)

Property: oil
$^1$H-NMR (DMSO-$d_6$, 100°C) δ: 2.25 (3H, s), 2.80-2.88 (2H, m), 2.93 (3H, s), 3.64-3.72 (2H, m), 6.71-7.15 (8H, m), 7.19-7.28 (1H, m), 7.33-7.40 (1H, m), 7.37 (1H, d, J=15.2Hz), 7.94-7.99 (1H, m), 8.49-8.53 (1H, m), 8.73-8.74 (1H, m)

Preparation example 48

Synthesis of (E)-N-(3-benzyloxyphenethyl)-3-(3-pyridyl)-2-propenamide (compound 82)

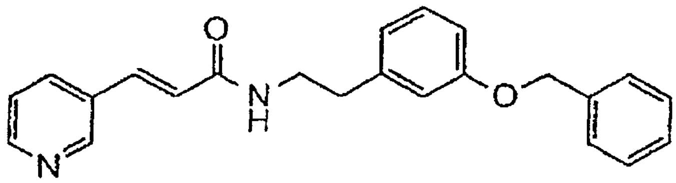

(82)

2.37 mL of triethylamine and 0.84 mL of pivaloyl chloride were added in order to a solution of 1.02 g of trans-3-(3-pyridyl)acrylic acid in 50 mL of dichloromethane while stirring on ice, and the solution was then stirred for 15 min. Subsequently, 1.59 g of 3-benzyloxyphenethylamine hydrochloride was added thereto at the same temperature, and the mixture was stirred for 1 h at room temperature. Water was added to the reaction mixture, the mixture was extracted using dichloromethane, washed with water, and then dried over magnesium sulfate. After removing the solvent by evaporation under reduced pressure, the residue was recrystallized from dichloromethane-hexane to obtain 1.70 g (79%) of the title compound.
Property: mp 115-116°C (dichloromethane-hexane)
$^1$H-NMR ($CDCl_3$)δ: 2.87 (2H, t, J=6.8Hz), 3.46-3.71 (2H, m), 5.06 (2H, s), 5.73 (1H, m), 6.37 (1H, d, J=15.7Hz), 6.81-6.89 (3H, m), 7.21-7.46 (7H, m), 7.61 (1H, d, J=15.7Hz), 7.73-7.79 (1H, m), 8.56 (1H, dd, J=4.8, 1.5Hz), 8.72 (1H, d, J=1.9Hz)

Preparation example 49

Synthesis of (E)-N-(3,4-dimethoxyphenethyl)-N-methyl-3-(3-pyridyl)-2-propenamide (compound 84)

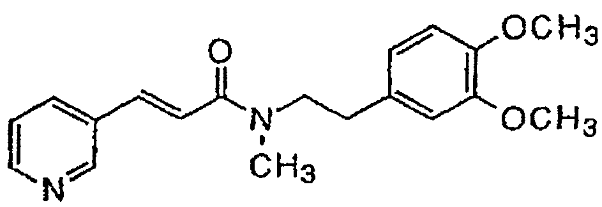

(84)

A mixture containing 326 mg of methyl trans-3-(3-pyridyl)acrylate, 390 mg of 3,4-dimethoxy-N-methylphenethylamine, 80 mg of 60% sodium hydride and 2 mL of diethylene glycol dimethyl ether was stirred at room temperature for 24 h. Water was added to the reaction mixture, the mixture was extracted with ethyl acetate, washed with water, and then dried over magnesium sulfate. The solvent was removed by vacuum evaporation and the resulting residual matter was purified by silica gel column chromatography (chloroform: methanol = 50:1) and then recrystallized to obtain 278 mg (43%) of the title compound.
Property: mp 84-86°C (ethyl acetate-hexane)
$^1$H-NMR (DMSO-$d_6$, 150°C) δ: 2.78 (2H, t, J=7.2Hz), 3.00 (3H, s), 3.67 (2H, t, J=7.2Hz), 3.69 (3H, s), 3.74 (3H, s), 6.72-6.75 (1H, m), 6.81-6.83 (2H, m), 6.95 (1H, d, J=15.6Hz), 7.31-7.36 (2H, m), 7.87-7.90 (1H, m), 8.48-8.50 (1H, m), 8.69-8.70 (1H, m)

Preparation example 50

Synthesis of methyl [3-[2-[(E)-3-(3-pyridyl) acryloylamino]ethyl]phenoxy]acetate (compound 94)

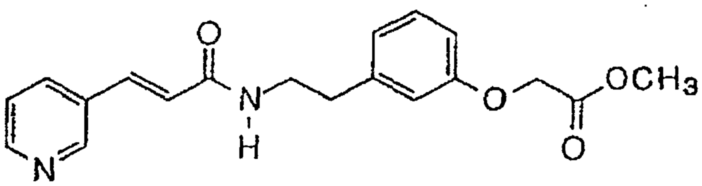

(94)

(1) Synthesis of (E)-N-(3-hydroxyphenethyl)-3-(3-pyridyl)-2-propenamide

584 mL of triethylamine and 148 mL of pivaloyl chloride were added in order to a solution containing 179 g of trans-3-(3-pyridyl)acrylic acid and 4.8 L of dichloromethane while stirring on ice, and the solution was stirred for 15 min. Subsequently, 263 g of 3-hydroxyphenethylamine hydrobromide was added under the same temperature, and the resulting mixture was stirred for 2 h. The solvent was removed by vacuum evaporation, and water was added to the residue. The precipitated crystals were filtered and washed with water. These were recrystallized with ethanol to obtain 251.4 g (78%) of the title compound.

Property: mp 163.0-164.5°C (ethanol)

$^1$H-NMR (DMSO-$d_6$)δ: 2.70 (2H, t, J=7Hz), 3.40 (2H, td, J=7, 5Hz), 6.59-6.66 (3H, m), 6.73 (1H, d, J=16Hz), 7.04-7.12 (1H, m), 7.39-7.45 (1H, m), 7.46 (1H, d, J=16Hz), 7.94-7.98 (1H, m), 8.24 (1H, t, J=5Hz), 8.52-8.56 (1H, m), 8.73-8.74 (1H, m), 9.25 (1H, s)

(2) 1.07 g (4.0 mmol) of (E)-N-(3-hydroxyphenethyl)-3-(3-pyridyl)-2-propenamide obtained in (1) and 0.52 g (4.8 mmol) of methyl chloroacetate was dissolved in 12 mL of dimethylformamide, 1.66 g (12 mmol) of potassium carbonate was added, and the resulting mixture was stirred for 8 h at 60°C. After standing to cool, ethyl acetate was added to the reaction mixture, insoluble matter was filtered off, the filtrate was washed with water and then dried over magnesium sulfate. After removing the solvent by evaporation under reduced pressure, the residue was recrystallized to obtain 0.83 g (61 %) of the title compound.

Property: mp 102-104°C (ethyl acetate)

$^1$H-NMR (DMSO-$d_6$)δ: 2.78 (2H, t, J=7Hz), 3.44 (2H, td, J=7, 6Hz), 3.71 (3H, s), 4.79 (2H, s), 6.74 (1H, d, J=16Hz), 6.76-6.88 (3H, m), 7.23 (1H, t, J=8Hz), 7.44-7.51 (1H, m), 7.47 (1H, d, J=16Hz), 7.99 (1H, d, J=8Hz), 8.27 (1H, t, J=6Hz), 8.57 (1H, dd, J=5, 1Hz), 8.77 (1H, d, J=2Hz)

Preparation example 51

Compound 105 was obtained by a method according to preparation example 50.

Compound 105

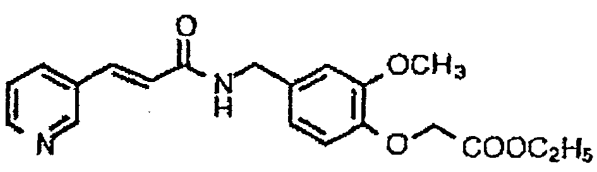

(105)

Property: mp 105-107°C (ethyl acetate)

$^1$H-NMR ($CDCl_3$)δ: 1.28 (3H, t, J=7.1Hz), 3.85 (3H, s), 4.25 (2H, q, J=7.1Hz), 4.51 (2H, d, J=5.7Hz), 4.66 (2H, s), 6.30-6.40 (1H, br), 6.49 (1H, d, J=15.7Hz), 6.77 (1H, d, J=8.1Hz), 6.81-6.89 (2H, m), 7.27-7.34 (1H, m), 7.65 (1H, d, J=15.7Hz), 7.74-7.80 (1H, m), 8.52-8.56 (1H, m), 8.68-8.69 (1H, m)

Preparation example 52

Synthesis of (E)-N-(3,4-dimethoxyphenethyl)-N-methyl-3-(3-pyridyl)-2-propenethioamide hydrochloride (compound 135)

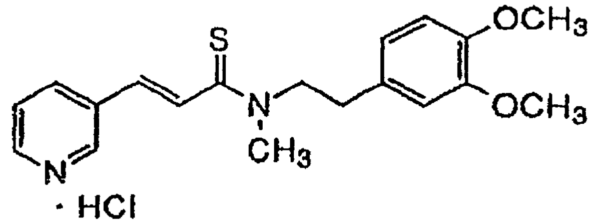

(135)

A mixture of 1.63 g of (E)-N-(3,4-dimethoxyphenethyl)-N-methyl-3-(3-pyridyl)-2-propenamide obtained in preparation example 49, 1.03 g of Lawesson's reagent and 10 mL of xylene was heated under reflux for 2 h. After removing the solvent by evaporation under reduced pressure, the resulting residual matter was purified by silica gel column chromatography (chloroform:methanol = 30:1) to obtain 1.66 g (97%) of (E)-N-(3,4-dimethoxyphenethyl)-N-methyl- 3-(3-pyridyl)-2-propenethioamide as oil. Next, after making this product into its hydrochloride by adding hydrogen chloride-methanol, it was recrystallized by a mixed solvent of ethyl acetate and methanol to obtain 1.68 g (89%) of the title compound.
Property: mp 167-169°C (ethyl acetate-methanol)
$^1$H-NMR (DMSO-$d_6$, 100°C) δ: 2.79 (2H, t, J=7.1Hz), 3.00 (3H, s), 3.67-3.74 (2H, m), 3.67(3H, s), 3.72 (3H, s), 6.70-6.83 (3H, m), 7.15 (1H, d, J=15.1Hz), 7.37 (1H, d, J=15.1Hz), 7.66-7.73 (1H, m), 8.33-8.37 (1H, m), 8.63-8.66 (1H, m), 8.93 (1H, br s)

Preparation example 53

Compound 136 was obtained by a method according to preparation example 52.

Compound 136

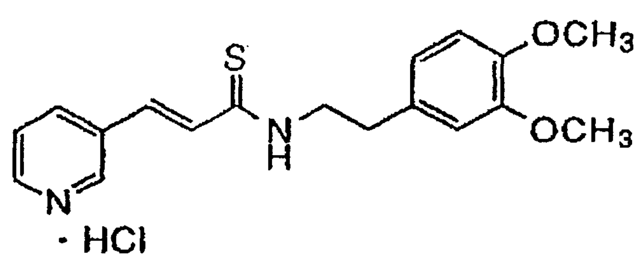

(136)

Property: mp 182-187°C (methanol)
$^1$H-NMR (DMSO-$d_6$)δ: 2.88-2.95 (2H, m), 3.72 (3H, s), 3.75 (3H, s), 3.81-3.92 (2H, m), 6.76-6.91 (3H, m), 7.44 (1H, d, J=15.6Hz), 7.77 (1H, d, J=15.6Hz), 7.96 (1H, dd, J=8.2, 5.4Hz), 8.58 (1H, d, J=8.2Hz), 8.82 (1H, d, J=5.4Hz), 9.08 (1H, br s), 10.62 (1H, t, J=5.2Hz)

Preparation examples 54 to 61

(E)-N-(3,4-dimethoxyphenethyl)-N-methyl-3-(3-pyridyl)-2-propenamide obtained in preparation example 49 was used as raw material and treated with inorganic acids or organic acids to obtain compound 140 to compound 147.

Preparation example 54

Compound 140

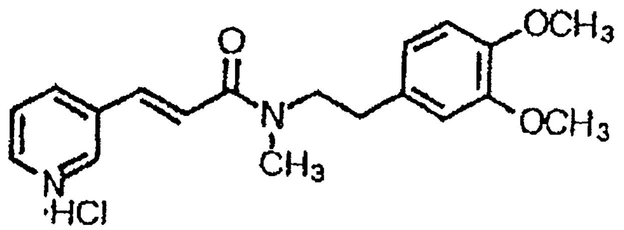

(140)

Property: mp 165-170°C (isopropanol)
$^1$H-NMR (DMSO-$d_6$, 100°C) δ: 2.78 (2H, t, J=7.1Hz), 3.00 (3H, s), 3.66 (3H, s), 3.66-3.72 (2H, m), 3.72 (3H, s), 6.70-6.84 (3H, m), 7.08 (1H, d, J=14.8Hz), 7.36 (1H, d, J=14.8Hz), 7.53-7.60 (1H, m), 8.17-8.22 (1H, m), 8.57-8.60 (1H, m), 8.84 (1H, s)

Preparation example 55

Compound 141

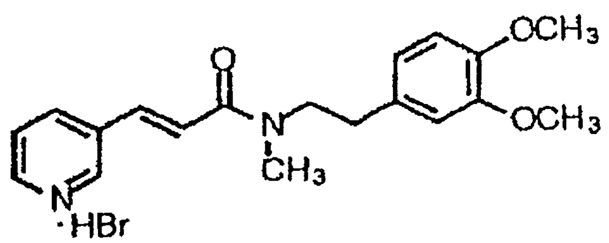

(141)

Property: mp 201-205°C (ether-methanol)
$^1$H-NMR (DMSO-$d_6$, 100°C) δ: 2.78 (2H, t, J=7.0Hz), 3.00 (3H, s), 3.66 (3H, s), 3.66-3.72 (2H, m), 3.72 (3H, s), 6.70-6.82 (3H, m), 7.11 (1H, d, J=15.6Hz), 7.37 (1H, d, J=15.6Hz), 7.60-7.67 (1H, m), 8.26-8.31 (1H, m), 8.61-8.65 (1H, m), 8.88 (1H, s)

Preparation example 56

Compound 142

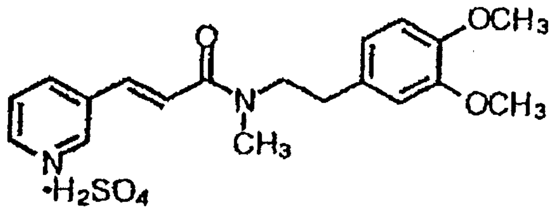

(142)

Property: mp 138°C (ether-methanol)
$^1$H-NMR (DMSO-$d_6$, 100°C) δ: 2.78 (2H, t, J=7.0Hz), 3.00 (3H, s), 3.66 (3H, s), 3.66-3.72 (2H, m), 3.72 (3H, s), 6.71-6.81 (3H, m), 7.07-7.16 (1H, m), 7.33-7.41 (1H, m), 7.63-7.71 (1H, m), 8.29-8.34 (1H,m), 8.62-8.66 (1H, m), 8.88 (1H, s)

Preparation example 57

Compound 143

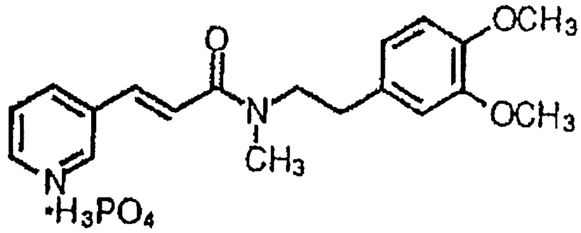

(143)

Property: mp 152°C (ether-methanol)
$^1$H-NMR (DMSO-$d_6$, 100°C) δ: 2.78 (2H, t, J=7.1Hz), 2.99 (3H, s), 3.63-3.71 (2H, m), 3.67 (3H, s), 3.72 (3H, s), 6.70-6.84 (3H, m), 6.96-7.04 (1H, m), 7.20-7.40 (2H, m), 7.93-7.98 (1H, m), 8.48-8.52 (1H, m), 8.72 (1H, s)

Preparation example 58

Compound 144

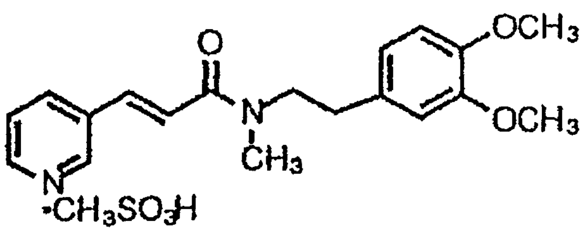

(144)

Property: amorphous
$^1$H-NMR (DMSO-$d_6$, 100°C) δ: 2.44 (3H, s), 2.78 (2H, t, J=7.1Hz), 3.00 (3H, s), 3.67 (3H, s), 3.66-3.72 (2H, m), 3.72 (3H, s), 6.71-6.84 (3H, m), 7.09 (1H, d, J=15.2Hz), 7.36 (1H, d, J=15.2Hz), 7.58-7.66 (1H, m), 8.23-8.28 (1H, m), 8.60-8.63 (1H, m), 8.85 (1H, s)

Preparation example 59

Compound 145

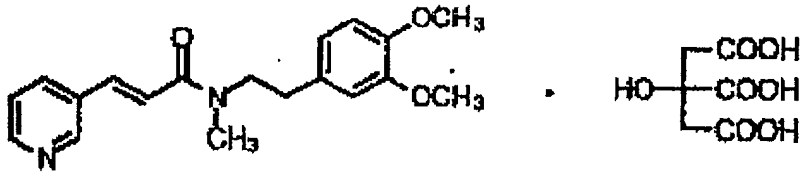

(145)

Property: mp 129.5-131.5°C (acetone)
$^1$H-NMR (DMSO-$d_6$, 100°C) δ: 2.71-2.82 (6H, m), 2.99 (3H, s), 3.63-3.71 (2H, m), 3.67 (3H, s), 3.72 (3H, s), 6.70-6.76 (1H, m), 6.79-6.84 (2H, m), 6.95-7.04 (1H, m), 7.29-7.40 (2H, m), 7.92-7.97 (1H, m), 8.48-8.52 (1H, m), 8.72 (1H, s)

Preparation example 60

Compound 146

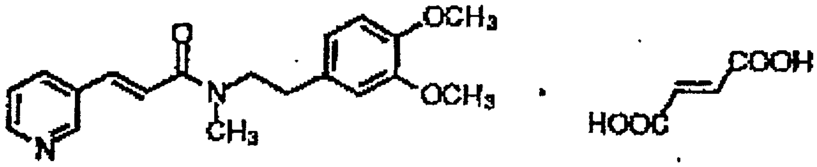

(146)

Property: mp 128.5-130°C (ethanol)
$^1$H-NMR (DMSO-$d_6$, 100°C) δ: 2.78 (2H, t, J=7.1Hz), 2.99 (3H, s), 3.67 (3H, s), 3.67 (2H, t, J=7.1Hz), 3.72 (3H, s), 6.63 (2H, s), 6.70-6.76 (1H, m), 6.80-6.85 (2H, m), 6.95-7.04 (1H, m), 7.29-7.39 (2H, m), 7.92-7.97 (1H, m), 8.48-8.52 (1H, m), 8.72 (1H, s)

Preparation example 61

Compound 147

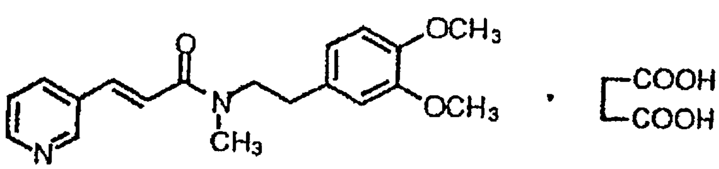

(147)

Property: mp 104-106°C (acetone)
$^1$H-NMR (DMSO-$d_6$, 100°C) δ: 2.43 (4H, s), 2.78 (2H, t, J=7.1Hz), 2.99 (3H, s), 3.63-3.72 (2H, m), 3.67 (3H, s), 3.72 (3H, s), 6.70-6.85 (3H, m), 6.95-7.04 (1H, m), 7.30-7.40 (2H,m), 7.92-7.97 (1H, m), 8.48-8.52 (1H, m), 8.73 (1H, s)

Preparation examples 62 and 63

Compound 153 and compound 154 were obtained by a method according to preparation example 3.

Preparation example 62

Compound 153

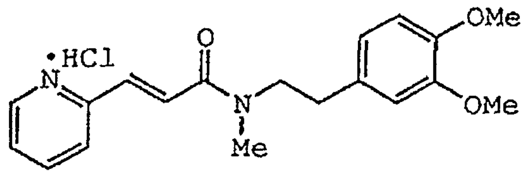

(153)

Property: mp 172-174°C (methanol-ether)
$^1$H-NMR (DMSO-$d_6$, 100°C) δ: 2.79 (2H, t, J=7.0Hz), 3.00 (3H, s), 3.66-3.72 (8H, m), 6.70-6.83 (3H, m), 7.30-7.50 (3H, m), 7.72-7.76 (1H, m), 7.94-8.02 (1H, m), 8.61-8.64 (1H, m)

Preparation example 63

Compound 154

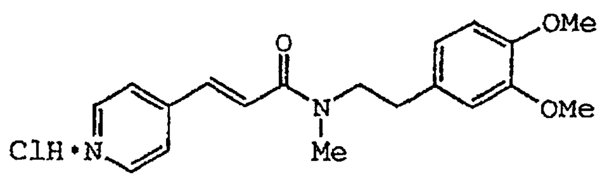

(154)

Property: mp 192-195°C (methanol-ether)
$^1$H-NMR (DMSO-$d_6$, 100°C) δ: 2.78 (2H, t, J=7.0Hz), 3.01 (3H, s), 3.65-3.71 (8H, m), 6.69-6.80 (3H, m), 7.29 (2H, m), 7.86-7.90 (2H, m), 8.70-8.73 (2H, m)

Preparation example 64

Synthesis of (E)-N-[2-(3,4-dimethoxyphenyl)-2-oxoethyl]-N-methyl-3-(3-pyridyl)-2-propenamide (compound 155)

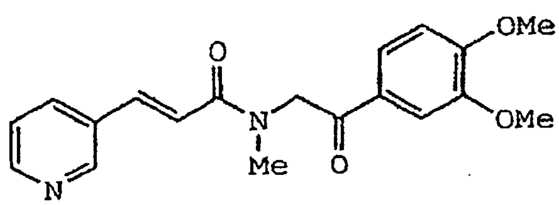

(155)

250 mL of ether and 100 mL of chloroform were added to 14.65 g (81 mmol) of 3',4'-dimethoxyacetophenone

and the mixture was stirred while cooling on ice. 4.1 mL of bromine was dissolved in 22 mL of chloroform, and added dropwise to the reaction mixture over 1 h. After stirring the reaction mixture for 1 h at room temperature, the reaction mixture was washed with water, aqueous saturated sodium bicarbonate, and water in that order. The organic phase was dried over magnesium sulfate and the solvent was then removed by vacuum evaporation. The residue was purified by silica gel column chromatography (dichloromethane: ethyl acetate = 30:1) to obtain 14.90 g (71%) of 2-bromo-1-(3,4-dimethoxyphenyl)ethanone.

$^1$H-NMR ($CDCl_3$)δ: 3.95 (3H, s), 3.97 (3H, s), 4.41 (2H, s), 6.91 (1H, d, J=8Hz), 7.55 (1H, d, J=2Hz), 7.62 (1H, dd, J=8Hz, 2Hz)

133 mL of 40% aqueous methylamine solution was added to 200 mL of isopropanol and stirred while cooling on ice. 8.47 g (33 mmol) of 2-bromo-1-(3,4-dimethoxyphenyl)ethanone was dissolved in 10 mL of isopropanol and 10 mL of dichloromethane, and the resulting solution was added dropwise to the above reaction mixture over 1 h. After the dropwise addition was completed the resulting mixture was stirred for 15 min while cooling on ice. The solvent of the reaction mixture was removed by vacuum evaporation at room temperature, and the precipitated crystals were filtered to obtain 6.36 g (67%) of 1-(3,4-dimethoxyphenyl)- 2-(methylamino)ethanone hydrobromide.

$^1$H-NMR ($CDCl_3$ + MeOH-$d_4$)δ: 2.81 (3H, s), 3.96 (3H, s), 3.98 (3H, s), 4.60 (2H, s), 6.99 (1H, d, J=8Hz), 7.53 (1H, d, J=2Hz), 7.64 (1H, dd, J=8Hz, 2Hz)

50 mL of dichloromethane and 2.69 mL (19.30 mmol) of triethylamine were added in order to 1.44 g (9.65 mmol) of trans-3-(3-pyridyl)acrylic acid and stirred for 10 min. Thereafter, 1.18 mL (9.65 mmol) of pivaloyl chloride was added and the solution was stirred for 13 min. 2.79 g (9.65 mmol) of 1-(3,4-dimethoxyphenyl)-2-(methylamino)ethanone hydrobromide was dissolved in a solution containing 4 mL of dichloromethane and 1.34 mL (9.65 mmol) of triethylamine, and the resulting solution was added to the reaction mixture and stirred at room temperature for 30 min. After washing the reaction mixture with water and aqueous saturated sodium bicarbonate, the organic phase was dried over magnesium sulfate, and the solvent was then removed by reduced-pressure evaporation. The residue was purified by silica gel column chromatography (dichloromethane: methanol = 10:1) to obtain a crude product. The crude product was recrystallized with dichloromethane/methanol/hexane to obtain 1.84 g (5.41 mmol, 56%) of the title compound.

Property: mp 193-194°C (dichloromethane/methanol/hexane)

$^1$H-NMR (DMSO-$d_6$, 100°C) δ: 2.95 (3H, s), 3.83 (3H, s), 3.87 (3H, s), 4.97 (2H, br), 7.09 (1H, d, J=8Hz), 7.26 (1H, br), 7.34 (1H, dd, J=8Hz, 5Hz), 7.48 (1H, d, J=15Hz), 7.51 (1H, d, J=2Hz), 7.65 (1H, dd, J=8Hz, 2Hz), 8.01 (1H, m), 8.49-8.52 (1H, m), 8.79 (1H, m)

Preparation example 65

Synthesis of (E)-N-[2-(3,4-dimethoxyphenyl)-2-(hydroxyimino)ethyl]-N-methyl-3-(3-pyridyl)-2-propenamide (compound 156)

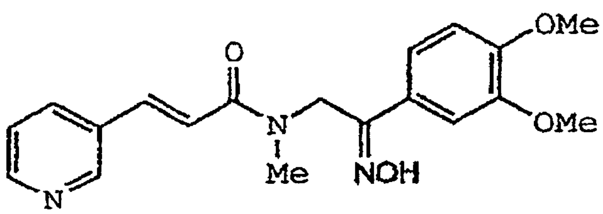

(156)

3 mL of acetic acid was added to 165 mg (0.5 mmol) of (E)-N-[2-(3,4-dimethoxyphenyl)-2-oxoethyl]-N-methyl-3-(3-pyridyl)-2-propenamide and left to stand at -20°C. After the acetic acid had solidified, 0.62 mL (10 mmol) of 50 % aqueous hydroxylamine solution was added while cooling in an ice-bath and the mixture was allowed to react at the same temperature. After returning the mixture to room temperature and reacting for a further 22 h, 10 mL of water and 10 mL of ethyl acetate were added, and mixture was extracted 3 times with 10 mL of ethyl acetate. The organic phase was washed with 40 mL of water and 40 mL of saturated saline solution in that order, and then dried over 10 g of anhydrous magnesium sulfate. The drying agent was then removed, and the filtrate concentrated. The thus obtained dry product was purified by column chromatography using 20 g of silica gel (eluant; dichloromethane: methanol = 100: 3.5). After purification, the residue was recrystallized from 5 mL of ethyl acetate and 15 mL of n-hexane. 91 mg (yield: 51 %) of the title compound was obtained.

Property: mp 172-173°C (ethyl acetate-hexane)

$^1$H-NMR (DMSO-$d_6$, 100°C) δ: 11.2 (1H, brs), 8.78-8.88 (1H, d), 8.50-8.53 (1H, dd, $J_1$=1.4 Hz, $J_2$=5.4Hz), 8.00-8.04

(1H, d, J=1.88Hz), 7.42-7.50 (1H, d), 7.33-7.40 (1H, m), 7.10-7.18 (2H, m), 7.18, 7.19 (1H, d, 4.4Hz), 6.89-6.93 (1H, d), 4.82 (2H, s), 3.75 (3H, s), 3.72 (3H, s), 2.91 (3H, s)

Preparation example 66

Synthesis of (E)-N-[2-hydroxy-2-(3-methoxy-4-hydroxyphenyl)ethyl]-N-methyl-3-(3-pyridyl)-2-propenamide (compound 158)

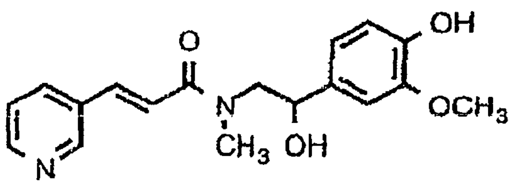

(158)

0.70 mL of pivaloyl chloride was added to a 10 mL dimethylformamide solution containing 805 mg of trans-3-(3-pyridyl) acrylic acid and 0.83 mL of triethylamine and stirred for 10 min at room temperature. Thereafter, a 10 mL dimethylformamide solution containing 1.26 g of metanephrine hydrochloride and 1.66 mL of triethylamine was added, and the resulting mixture was stirred at room temperature for 1 h. Water was added to the reaction mixture, and the mixture was extracted with ethyl acetate, washed with water, and then dried over magnesium sulfate. After removing the solvent by evaporation under reduced pressure, the residue was purified by silica gel column chromatography (dichloromethane: methanol = 50:1) to obtain 1.41 g (80%) of the title compound.
Property: amorphous
$^1$H-NMR (DMSO-$d_6$, 150°C) δ: 3.00(3H,s), 3.47-3.70 (2H, m), 3.76 (3H, s), 4.66-4.91 (2H, m), 6.71 (1H, d, J=8.0Hz), 6.78 (1H, dd, J=8.0, 1.8Hz), 6.93 (1H, d, J=1.8Hz), 7.00 (1H, d, J=15.6Hz), 7.25-7.42 (1H, m), 7.34 (1H, d, J=15.6Hz), 7.79-8.02 (2H, m), 8.49 (1H, dd, J=4.8, 1.6Hz), 8.71 (1H, d, J=2.2Hz)

Preparation example 67

Synthesis of (E)-N-methyl-N-(3-methoxy-4-hydroxyphenacyl)-3-(3-pyridyl)-2-propenamide (compound 162)

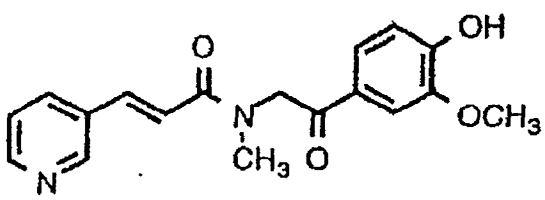

(162)

468 mg (2 mmol) of 2,3-dichloro-5,6-dicyano-1,4-benzoquinone was added to a 12 mL dioxane solution containing 656 mg (2 mmol) of (E)-N-[2-hydroxy-2-(3-methoxy-4-hydroxyphenyl)ethyl]-N-methyl-3-(3-pyridyl)-2-propenamide under an argon atmosphere, and stirred at room temperature for 2 h. The precipitated crystals were filtered off, and the solvent then removed by evaporation under reduced pressure. The residue was purified by column chromatography (chloroform: methanol = 50:1) and then dried under vacuum to obtain 362 mg (56 %) of the title compound.
Property: amorphous
$^1$H-NMR (DMSO-$d_6$, 100°C) δ: 3.13 (3H, brs), 3.88 (3H, s), 4.97 (2H, brs), 6.87-6.99 (1H, m), 7.04-7.62 (5H, m), 7.93-8.17 (1H, m), 8.43-8.64 (1H, m), 8.70-8.95 (1H, m), 9.59 (1H, brs)

Preparation example 68

Compound 163 was obtained by a method according to preparation example 64.

Compound 163

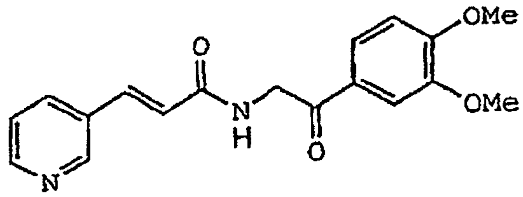

(163)

Property: solid

$^1$H-NMR ($CDCl_3$)δ: 3.97 (3H, s), 3.98 (3H, s), 4.88 (2H, d, J=4.2Hz), 6.66 (1H, d, J=15.7Hz), 6.90 (1H, brs), 6.95 (1H, d, J=8.5Hz), 7.34 (1H, dd, J=7.9, 4.8Hz), 7.54 (1H, d, J=2.0Hz), 7.69 (1H, d, J=15.9Hz), 7.70 (1H, d, J=2.0Hz), 7.83-7.85 (1H, m), 8.59 (1H, dd, J=4.8, 1.4Hz), 8.78 (1H, d, J=1.8Hz)

Preparation example 69

Synthesis of (E)-N-[2-(3,4-dimethoxyphenyl)-2-(methylthio)ethyl]-3-(3-pyridyl)-2-propenamide (compound 164)

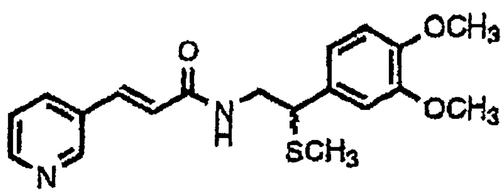

(164)

1.42 g (8 mmol) of trans-3,4-dimethoxy-β-nitrostyrene was added to a solution of 0.62 g (8.8 mmol) of sodium methanethiolate in 20 mL of methanol and stirred for 5 min at room temperature. Thereafter, 0.46 mL of acetic acid was added and the resulting solution stirred for a further 5 min. After concentrating the methanol to moiety under reduced pressure, water was added, and the mixture then extracted with dichloromethane. The organic phase was collected and washed with water, and then dried over sodium sulfate. The solvent was removed by evaporation under reduced pressure, and the resulting residue was separated and purified by silica gel column chromatography (hexane: ethyl acetate = 10:1) to obtain 1.24 g (60%) of 2-(3,4-dimethoxyphenyl)-2-(methylthio) nitroethane.

A 20 mL tetrahydrofuran solution containing 1.22 g (4.8 mmol) of 2-(3,4-dimethoxyphenyl)-2-(methylthio)nitroethane was added dropwise under an argon atmosphere to a 10 mL tetrahydrofuran solution containing 0.47 g of lithium aluminum hydride that was being stirred on ice. After stirring at room temperature for 30 min, 0.47 mL of water, 0.47 g of 15% aqueous sodium hydroxide, and 1.14 mL of water were added dropwise in order to the reaction mixture while stirring on ice. A small amount of potassium carbonate was added and the resulting mixture was stirred for several minutes. Thereafter, the inorganic salts were filtered off and washed with tetrahydrofuran. The filtrate was concentrated under vacuum and then dried to obtain 0.98 g of 2-(3,4-dimethoxyphenyl)-2-(methylthio)ethylamine as crude oil.

0.69 mL of diethyl phosphorocyanidate and 1.17 mL of triethylamine were added in order to a 10 mL dimethylformamide solution containing 0.96 g of 2-(3,4-dimethoxyphenyl)-2-(methylthio)ethylamine as crude oil and 0.63 g (4.2 mmol) of trans-3-(3-pyridyl)acrylic acid while cooling on ice, and the resulting solution was stirred for 10 min while ice-cooling. Aqueous sodium bicarbonate was added to the reaction solution, and the solution was then extracted with ethyl acetate. The organic phase was collected, washed with water and saturated saline solution, and then dried over magnesium sulfate. The solvent was removed by vacuum evaporation and the residue was purified by silica gel column chromatography (hexane: chloroform: ethanol = 8:2:1) to obtain 788 mg (47% ) of the title compound. Property: amorphous

$^1$H-NMR ($CDCl_3$)δ: 2.00 (3H, s), 3.64-3.99 (3H, m), 3.87 (3H, s), 3.88 (3H, s), 6.06-6.30 (1H, m), 6.43 (1H, d, J=15.8Hz), 6.74-6.97 (3H, m), 7.29 (1H, dd, J=8.0, 4.8Hz), 7.61 (1H, d, J=15.8Hz), 7.70-7.86 (1H, m), 8.55 (1H, dd, J=4.8, 1.6Hz), 8.69 (1H, d, J=2.0Hz)

Preparation example 70

Synthesis of (E)-N-[2-(3,4-dimethoxyphenyl)-2-oxoethyl]-N-methyl-3-(3-pyridyl)-2-propenethioamide (compound 165)

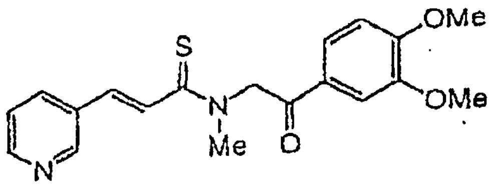

(165)

300 mg of Lawesson's reagent and 20 mL of anhydrous toluene were added to 390 mg of the compound obtained in preparation example 64, and the mixture was refluxed under an argon atmosphere. After 4 h, 30 mL of ethyl acetate and 30 mL of water were added, whereby the ethyl acetate phase was separated, and then extracted 2 times from the aqueous phase with a further 20 mL of ethyl acetate. The ethyl acetate phase was combined, washed with 50 mL of water and 50 mL of saturated saline solution in order, and dried over anhydrous magnesium sulfate. The solvent was removed by evaporation, and the residue was purified by silica gel column chromatography (dichloromethane: methanol = 1000:15) to obtain 61 mg (14%) of the title compound.
Property: solid
$^1$H-NMR (DMSO-$d_6$)$\delta$: 3.53 (3H, s), 3.94 (3H, s), 3.97 (3H, s), 5.60 (2H, s), 6.93 (1H, d, J=15Hz), 7.35 (1H, d, J=15Hz), 7.55 (1H, d, J=1.9Hz), 7.63-7.82 (1H, m), 7.78-7.87 (1H, m), 8.49-8.67 (3H, m), 8.80 (1H, d, J=2.0Hz)

Example Inhibitory action on phosphodiesterase IV

Phosphodiesterase IV was isolated using U-937 cell as origin (Torphy, T. J. et al., J. Pharmacol. Exp. Ther., 263, 1195-1205 (1992)). Using [$^3$H]cAMP and CAMP (1 μM) as substrate/tracer, incubation was performed for 30 min at 30°C. [$^3$ H]5'-AMP was measured by liquid scintillation. Inhibitory activity is represented by the rate of inhibition of a test substance with respect to a control group that is without the test substance, and is calculated by the following formula:

Inhibitory activity (%) = 100 × (control group value - value of group with test substance addition)/control group value.

All tests were implemented in duplicate. The results are shown in Table 1.

Table 1

| Compound No. | Phosphodiesterase IV inhibitory activity (10 μM) |
|---|---|
| 2 | 72 |
| 12 | 86 |
| 16 | 83 |
| 20 | 73 |
| 29 | 47 |
| 30 | 59 |
| 63 | 40 |
| 84 | 49 |
| 135 | 53 |
| 136 | 45 |
| 140 | 54 |
| 143 | 50 |

Table 1 (continued)

| Compound No. | Phosphodiesterase IV inhibitory activity (10 μM) |
|---|---|
| 144 | 47 |
| 146 | 43 |
| 153 | 59 |
| 154 | 58 |
| 155 | 69 |

All publications, patents and patent applications cited herein are incorporated herein by reference in their entirety.

Industrial Applicability

According to the present invention, there can be provided a phosphodiesterase IV inhibitor containing a pyridylacrylamide derivative as an active ingredient.

## Claims

**1.** A phosphodiesterase IV inhibitor comprising as an active ingredient a pyridylacrylamide derivative represented by the following formula (I):

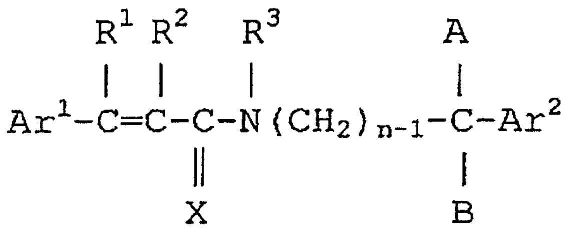

(I)

wherein

Ar$^1$ represents a substituted or unsubstituted pyridyl group;
Ar$^2$ represents a substituted phenyl group that is substituted with at least 1 to 3 substituents selected from the group consisting of a $C_{1-6}$ alkoxy group, a $C_{2-6}$ alkenyloxy group, an aralkyloxy group, and an aryloxy group;
R$^1$ represents a hydrogen atom, a $C_{1-6}$ alkyl group, or an aryl group;
R$^2$ represents a hydrogen atom, a $C_{1-6}$ alkyl group, a cyano group, or a $C_{1-6}$ alkoxy-carbonyl group;
R$^3$ represents a hydrogen atom or an optionally substituted $C_{1-6}$ alkyl group;
X represents an oxygen atom or a sulfur atom;
A and B are the same or different from each other, and each independently represents a hydrogen atom, a hydroxyl group, a $C_{1-6}$ alkoxy group or a $C_{1-6}$ alkylthio group, or A and B together represent an oxo group, a thioxo group, a group represented by the following formula:

$$= N\text{-}Y$$

wherein Y represents a di-($C_{1-6}$ alkyl) amino group, a hydroxyl group, an aralkyloxy group, or a $C_{1-6}$ alkoxy group, or a group represented by the following formula:

$$-Z^1\text{-}M\text{-}Z^2\text{-}$$

wherein, $Z^1$ and $Z^2$ are the same or different from each other, and each independently represents an oxygen atom, a sulfur atom, or an imino group that may be optionally substituted with a $C_{1-6}$ alkyl group; and M represents an alkylene group having 2 to 4 chain members or a 1,2-phenylene group, or
A may be a hydroxyl group and B may be a 1-$C_{1-6}$ alkyl-imidazol-2-yl group; and
n represents an integer from 1 to 3,

or a pharmaceutically acceptable salt thereof.

**2.** The phosphodiesterase IV inhibitor according to claim 1, wherein in the formula (I), $Ar^1$ represents a substituted or unsubstituted pyridyl group; $Ar^2$ represents a substituted phenyl group that is substituted with at least 1 to 3 substituents selected from the group consisting of a $C_{1-6}$ alkoxy group, a $C_{2-6}$ alkenyloxy group, an aralkyloxy group, and an aryloxy group; R' represents a hydrogen atom, a $C_{1-6}$ alkyl group, or an aryl group; $R^2$ represents a hydrogen atom, a methyl group, a cyano group, or a $C_{1-6}$ alkoxy-carbonyl group; $R^3$ represents a hydrogen atom or an optionally substituted $C_{1-3}$ alkyl group; X represents an oxygen atom or a sulfur atom; A and B each independently represents a hydrogen atom, or A and B together represent an oxo group; provided that when A and B each independently represents a hydrogen atom, then n represents 1 or 2, and when A and B together represent an oxo group, then n represents 2.

**3.** The phosphodiesterase IV inhibitor according to claim 2, wherein in the formula (I), $Ar^2$ represents a substituted phenyl group that is substituted with 1 to 3 $C_{1-6}$ alkoxy groups, and $R^3$ represents a $C_{1-3}$ alkyl group.

**4.** The phosphodiesterase IV inhibitor according to claim 1, wherein in the formula (I), a substituted phenyl group represented by $Ar^2$ is further substituted with at least one member selected from the group consisting of a halogen atom, a hydroxyl group, an optionally substituted amino group, a substituted $C_{1-6}$ alkoxy group, an optionally substituted $C_{1-6}$ alkyl group, an aryl group, a $C_{1-6}$ alkylthio group, a carboxyl group, a $C_{1-6}$ alkoxy-carbonyl group, a sulfamoyl group and a group -O-CO-$R^4$ (where $R^4$ represents a $C_{1-6}$ alkyl group, an aryl group, a $C_{1-6}$ alkoxy group, or an optionally substituted amino group).

**5.** The phosphodiesterase IV inhibitor according to claim 1, which is a preventive or therapeutic agent for a phosphodiesterase IV-involving disease selected from the group consisting of bronchial asthma, chronic bronchitis, atopic dermatitis, hives, allergic rhinitis, conjunctivitis, rheumatoid arthritis, gonarthrosis, septicemia, ulcerative colitis, manic-depressive psychosis, schizophrenia and Crohn's disease.

# INTERNATIONAL SEARCH REPORT

International application No.
PCT/JP03/04227

A. CLASSIFICATION OF SUBJECT MATTER
Int.Cl[7] A61K31/4406, 31/443, 31/44, 31/4402, 31/4409, A61P1/04, 11/06, 11/08, 17/00, 19/02, 25/18, 25/24, 27/02, 27/16, 29/00, 31/04, 37/08, 43/00, 17/04//C07D213/56, 213/57,

According to International Patent Classification (IPC) or to both national classification and IPC

B. FIELDS SEARCHED

Minimum documentation searched (classification system followed by classification symbols)
Int.Cl[7] A61K31/4406, 31/443, 31/44, 31/4402, 31/4409, A61P1/04, 11/06, 11/08, 17/00, 19/02, 25/18, 25/24, 27/02, 27/16, 29/00, 31/04, 37/08, 43/00, 17/04//C07D213/56, 213/57,

Documentation searched other than minimum documentation to the extent that such documents are included in the fields searched

Electronic data base consulted during the international search (name of data base and, where practicable, search terms used)
REGISTRY(STN), CAPLUS(STN)

C. DOCUMENTS CONSIDERED TO BE RELEVANT

| Category* | Citation of document, with indication, where appropriate, of the relevant passages | Relevant to claim No. |
|---|---|---|
| A | WO 99/05109 A1 (TSUMURA & CO.), 04 February, 1999 (04.02.99), Full text & US 6313153 A | 1-5 |
| A | EP 882714 A1 (SS PHARMACEUTICAL CO., LTD.), 09 December, 1998 (09.12.98), Full text & WO 98/13348 A1 & US 5935977 A | 1-5 |
| A | WO 98/45268 A (PFIZER PROD INC.), 15 October, 1998 (15.10.98), Full text & JP 2000-510481 A & EP 0971894 A1 | 1-5 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07 July, 2003 (07.07.03) | 22 July, 2003 (22.07.03) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP03/04227

C (Continuation). DOCUMENTS CONSIDERED TO BE RELEVANT

| Category* | Citation of document, with indication, where appropriate, of the relevant passages | Relevant to claim No. |
|---|---|---|
| A | WO 93/04035 A1 (BOEHRINGER MANNHEIM GMBH), 04 March, 1993 (04.03.93), Full text & JP 6-510030 A & EP 0527458 A1 | 1-5 |

Form PCT/ISA/210 (continuation of second sheet) (July 1998)

**INTERNATIONAL SEARCH REPORT**

International application No.
PCT/JP03/04227

Continuation of A. CLASSIFICATION OF SUBJECT MATTER
(International Patent Classification (IPC))

Int.Cl$^7$ 213/59, 405/12, 213/61, 213/80, 213/65

(According to International Patent Classification (IPC) or to both national classification and IPC)

Continuation of B. FIELDS SEARCHED
Minimum Documentation Searched(International Patent Classification (IPC))

Int.Cl$^7$ 213/59, 405/12, 213/61, 213/80, 213/65

Minimum documentation searched (classification system followed by classification symbols)

Form PCT/ISA/210 (extra sheet) (July 1998)